# EUROPEAN PATENT APPLICATION

(11) **EP 3 827 825 A1**
(43) Date of publication of application: **02.06.2021**
(21) Application number: 19211619.2
(22) Date of filing: 26.11.2019
(51) Int. Cl.: A61K 31/415, A61K 31/42, A61P 31/04

(54) **USE OF 3, 4-DIARYLPYRAZOLES AS ANTIBACTERIAL AGENTS**

(71) Applicant: Wageningen Universiteit, 6708 PB Wageningen (NL)
(72) Inventor: WELLS, Jerry, 3703 DC ZEIST (NL); VELIKOVA, Nadya, 6708 WD WAGENINGEN (NL)
(74) Representative: EP&C

(57) **Abstract**

The use of a compound of formula (I) or a pharmaceutically acceptable salt or solvate thereof as an antibacterial or antibiotic 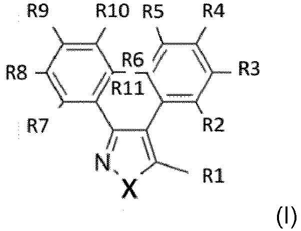 against *Staphylococcus, Escherichia, Streptococcus, Klebsiella, Mycobacterium, Pseudomonas, Clostridium, Acinetobacter, Enterococcus, Pasteurella Bacillus anthracis. Listeria monocytogenes, Corynebacterium diphtheriae, Salmonella, Shigella, Moraxella, Helicobacter, Legionella, Vibrio, Rickettsia, Neisseria, Actinobacter,* is disclosed.

## Description

### Background of the invention

The emergence of antibiotic resistance is one of the greatest challenges facing our society today. A recent survey estimated that if no new antimicrobial therapies are brought to the market in 2050, 10 million people will die worldwide every year as a result of infections caused by bacteria resistant to current antibiotics. The discovery and development of new antibiotics is therefore urgently needed (1). The aging population and growing number of immunocompromised patients due to HIV, cancer therapy or transplantation have increased the population susceptibility to bacterial infections making the need for novel antibacterials even more urgent. The so-called "ESKAPE" pathogens (*Enterococcus faecium, Staphylococcus aureus, Klebsiella pneumoniae, Acinetobacter baumannii, Pseudomonas aeruginosa, Enterobacterspp.*) are the main cause of hospital infections and are resistant to virtually all currently marketed antibiotics (2).

Bacterial two-component systems (TCS) are considered promising targets for the discovery of novel antibacterials as they are found in nearly all bacteria, have a new mechanism of action and lower potential of resistance development in comparison with conventional antibiotics (3). TCS are signal transduction devices used by nearly all bacteria that regulate a variety of processes including bacterial growth, cell-wall metabolism, virulence, biofilm formation and resistance to antibiotics (3, 4). In Firmicutes (which includes MRSA, VRE, and some other notorious pathogens) the WalKR TCS (a.k.a., YycGF, VicKR, MicAB) is an obligate essential regulatory system. Other single TCSs might not be essential for growth *in vitro,* but in vivo they can be necessary for survival or persistence (4). A prototypical TCS consist of a membrane bound histidine kinase (HK) and its cognate response regulator (RR). Upon sensing an environmental stimulus, the HK is autophosphorylated on conserved histidine residues in the dimerization and histidine phosphotransfer (DHp) domain by an ATP molecule binding to the catalytic and ATP-binding (CA) domain (reviewed in 3). Subsequently, the phosphoryl group from the conserved histidine is transferred to a conserved aspartic acid residue in the receiver (REC) domain of the RR. The phosphorylated state of the RR affects its binding affinity to a cognate DNA motif and/ or other protein partners, thereby modulating transcription of target genes (reviewed in 3). Putative HK autophosphorylation inhibitors (HKAIs) targeted at the CA domains of HKs were shown to inhibit multiple TCS due to the conservation between the HK CA domains (5). The conservation of the HK CA domains is hypothesized to determine the broad-spectrum antibacterial effect of the HKAIs (5). The reported putative HKAI were not active against most Gram-negative ESKAPE pathogens at concentrations as high as 500 µg/ml. However, when delivered via nanoparticles engineered to interact with the surface envelope of Gram-negative bacteria, the minimal inhibitory concentration against Gram negative *E. coli* CFT073 was lowered more than 20 times (6).

Vo et al. described certain 3, 4-diarylpyrazoles and their use as antibiotics targeting histidine kinase (7). There remains a need however for further development of this technical area.

### Summary of the invention.

The present invention concerns a group of compounds that have a 3, 4-diaryl-(ox- or pyr-(azole) backbone in common to be used as antibacterials and/or antibiotics. The compounds have found to be active against a wide group of pathogens such as *Staphylococcus, Escherichia, Streptococcus, Klebsiella, Mycobacterium, Pseudomonas, Clostridium, Acinetobacter, Enterococcus* and *Pasteurella*. The invention further relates to the use of the compounds as a medicament, antibacterial, antibiotic or in a pharmaceutical composition.

Thus, the invention pertains to the use of a compound of formula (I) or a pharmaceutically acceptable salt or solvate thereof as an antibacterial 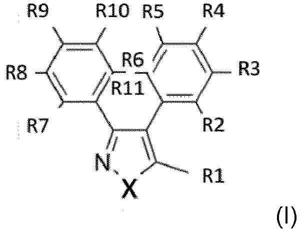 wherein
- **X** is: NH or O;
- **R1** is: H, C₁-C₄ alkyl, optionally substituted with one or more halogens, wherein the halogen is selected from F, Cl, Br;
- **R2** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R3** is: H, F, Cl, Br, C₁-C₄ alkyl optionally substituted with one or more halogens, selected from F, Cl, Br, C₁-C₄ alkoxy, optionally substituted with one or more halogens, selected from F, Cl, Br;
- **R4** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R5** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R6** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R7** is: H, OR12;
- **R8** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R9** is: H, OR13;
- **R10** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R11** is: H, halogen selected from F, Cl, Br, C₁-C₄ alkyl optionally substituted with one or more halogens, selected from F, Cl, Br, C₁-C₄ alkoxy optionally substituted with one or more halogens, selected from F, Cl, Br;
- **R12** is: H, C₁-C₄ alkyl;
- **R13** is: H , C₁-C₈ alkyl , C₂-C₈ alkenyl, CH2Ph, the phenyl ring optionally being substituted with one or more of C₁-C₄-alkyl, C₂-C₄-alkenyl, NO2, halogens selected from F, Cl, Br;

The compounds of the invention typically share a 3, 4-diaryl-(ox- or pyr-(azole) backbone.

The present compounds distinguish themselves over compounds having a similar backbone by having a higher activity and/or specific activity or selectivity against specific pathogens and/or have an improved resistance development.

In a preferred embodiment,
- **X** is: NH
- **R1** is: H, C₁-C₂ alkyl, optionally substituted with one or more halogens, wherein the halogen is selected from F, Cl, Br
- **R2** is: H, halogen selected from F, Cl, Br, C₁-C₂-alkyl, C₁-C₂ alkoxy
- **R3** is: H, Cl, Br, C₁-C₂ alkoxy
- **R4** is: H, halogen selected from F, Cl, Br, C₁-C₄ alkoxy
- **R5** is: H, halogen selected from F, Cl, Br, C₁-C₄ alkyl, C₁-C₄ alkoxy
- **R6** is: H, halogen selected from F, Cl, Br, C₁-C₄ alkyl, C₁-C₄ alkoxy
- **R7** is: H, OR12
- **R8** is: H, halogen selected from F, Cl, Br, C₁-C₄ alkyl, C₁-C₄ alkoxy
- **R9** is: H, OR13
- **R10** is: H, halogen selected from F, Cl, Br, C₁-C₄ alkyl, C₁-C₄ alkoxy
- **R11** is: H, halogen selected from F, Cl, Br, C₁-C₄ alkyl, C₁-C₄ alkoxy
- **R12** is: H, C₁-C₂-alkyl
- **R13** is: H, C₁-C6 alkyl, C₂-C6-alkenyl, phenyl ring being substituted with one or more of C₁-C₂-alkyl, C₁-C₄-alkenyl, NO2, halogens selected from F, Cl, Br

In a further preferred embodiment,
- **X** is: NH
- **R1** is: H, C₁ alkyl optionally substituted with one or more halogens, wherein the halogen is selected from F, Cl, Br
- **R2** is: H, Cl, C₁-alkyl, C₁-alkoxy
- **R3** is: H, Cl, Br, C₁-alkoxy
- **R4** is: H, halogen selected from F, Cl, Br, C₁-C₂ alkoxy
- **R5** is: H, Cl, Br, C₁-C₂-alkyl, C₁-C₂ alkoxy
- **R6** is: H, Cl, Br, C₁-C₂-alkyl, C₁-C₂ alkoxy
- **R7** is: H, OR12
- **R8** is: H, Cl, Br, C₁-C₂-alkyl, C₁-C₂ alkoxy
- **R9** is: H, OR13
- **R10** is: H, Cl, Br, C₁-C₂-alkyl, C₁-C₂ alkoxy
- **R11** is: H, Cl, Br, C₁-C₂-alkyl, C₁-C₂ alkoxy
- **R12** is: H,
- **R13** is: H, C₁-C₂-Alkyl, phenyl ring being substituted with one or more of C₁-alkyl, C₂-alkenyl, NO₂, halogens selected from F, Cl, Br,

The compounds are detailed with their structural formula, SMILES representation and systematic name in Table 1.

The compounds used in the present invention can be represented by their respective SMILES. The Simplified Molecular-Input Line-Entry System (SMILES) is a specification in the form of a line notation for describing the structure of chemical species using short ASCII strings. SMILES strings can be imported by most molecule editors for conversion back into two-dimensional drawings or three-dimensional models of the molecules.

SMILES representation is a generally known and accepted representation of chemical compounds. A common application of SMILES is indexing and ensuring uniqueness of molecules in a database.

The invention further pertains to the use of the compounds having the following smiles representation:
- OC1ccc(-C2[nH]nc(C2-C2ccc(Br)cC2)C(F)(F)F)c(O)C1;
- OC1ccc(-C2onc(C2-C2ccc(Cl)cC2)C(F)(F)F)c(O)C1;
- CC1noc(C1-C1ccc(Br)cC1)-C1ccc(O)cC1O;
- CC1[nH]nc(C1-C1ccc(Br)cC1)-C1ccc(O)cC1O;
- COC1ccc(cC1OC)-C1c([nH]nC1C(F)(F)F)-C1ccc(O)cC1O;
- CCOC1ccc(-C2[nH]nc(C2-C2ccc(OC)c(OC)C2)C(F)(F)F)c(O)C1;
- COC1ccc(-C2[nH]nc(C)C2-C2ccc(Br)cC2)c(O)C1;
- COC1ccc(-C2[nH]nc(C2-C2ccc(OC)c(OC)C2)C(F)(F)F)c(O)C1;
- CCOC1ccc(-C2[nH]nc(C2-C2ccc(Br)cC2)C(F)(F)F)c(O)C1;
- CCOC1ccc(-C2n[nH]c(C2-C2ccc(OC)cC2)C(F)(F)F)c(O)C1;
- COC1ccc(cC1)-C1c(n[nH]C1C(F)(F)F)-C1ccc(OC)cC1O;
- COC1ccc(cC1)-C1c(n[nH]C1C(F)(F)F)-C1ccc(O)cC1O;
- OC1ccc(-C2[nH]nc(C2-C2ccccC2)C(F)(F)F)c(O)C1;
- OC1cc(OCC2ccc(cC2)[N+]([O-])=O)ccC1-C1[nH]nc(C1-C1ccc(Br)cC1)C(F)(F)F;
- NNC(=O)COC1ccc(-C2[nH]nc(C2-C2ccc(Br)cC2)C(F)(F)F)c(O)C1;
- COC1ccccC1-C1c([nH]nC1C(F)(F)F)-C1ccc(O)cC1O;
- OC1ccc(-C2n[nH]c(C2-C2ccc(Cl)cC2)C(F)(F)F)c(O)C1;
- OC1cc(OCC2ccccC2)ccC1-C1n[nH]c(C1-C1ccc(Br)cC1)C(F)(F)F;
- COC1ccc(-C2n[nH]c(C2-C2ccc(Cl)cC2)C(F)(F)F)c(O)C1;
- COC1ccc(cC1)-C1c(n[nH]C1C(F)(F)F)-C1ccc(OCC(=O)NN)cC1O;
- OC1cc(OCC2ccc(Br)cC2)ccC1-C1[nH]nc(C1-C1ccc(Cl)cC1)C(F)(F)F;
- COC1ccc(-C2n[nH]c(C2-C2ccccC2OC)C(F)(F)F)c(O)C1;
- CCOC1ccc(-C2n[nH]c(C2-C2ccccC2OC)C(F)(F)F)c(O)C1;
- COC1ccc(-C2[nH]nc(C2-C2ccccC2)C(F)(F)F)c(O)C1;
- CCOC1ccc(-C2n[nH]c(C2-C2ccccC2)C(F)(F)F)c(O)C1;
- COC1ccc(-C2[nH]nc(C2-C2ccc(Br)cC2)C(F)(F)F)c(O)C1;
- OC1cc(OCC2ccccC2)ccC1-C1[nH]nc(C1-C1ccccC1)C(F)(F)F;
- NNC(=O)COC1ccc(-C2[nH]nc(C2-C2ccccC2)C(F)(F)F)c(O)C1;
- COC1ccc(cC1)-C1c(n[nH]C1-C1ccc(OCC2ccccC2)cC1O)C(F)(F)F;
- CCOC1ccc(-C2[nH]nc(C2-C2ccc(Cl)cC2)C(F)(F)F)c(O)C1;
- OC1ccc(-C2n[nH]c(C2-C2ccc(F)cC2)C(F)(F)F)c(O)C1;
- COC1ccc(cC1)-C1c(n[nH]C1C(F)(F)F)-C1ccc(OC)c(C)C1O;
- CCOC1cc(O)c(cC1CC)-C1n[nH]c(C1-C1ccccC1)C(F)(F)F;
- CCOC1ccc(-C2n[nH]c(C2-C2ccccC2)C(F)(F)F)c(O)C1C;
- CC1ccc(COC2ccc(-c3n[nH]c(c3-c3cccC4ccccc34)C(F)(F)F)c(O)C2)cC1;
- CCCCCOC1ccc(-C2n[nH]c(C2-C2ccccC2Cl)C(F)(F)F)c(O)C1;
- COC1ccc(cC1)-C1c(n[nH]C1-C1ccc(OCC2ccc(cC2)[N+]([O-])=O)cC1O)C(F)(F)F;
- COC1ccc(cC1)-C1c(n[nH]C1-C1ccc(OCC(C)=C)cC1O)C(F)(F)F;
- COC1ccc(cC1)-C1c(n[nH]C1-C1ccc(OCC2ccccC2F)cC1O)C(F)(F)F;
- CC(=C)COC1ccc(-C2[nH]nc(C2-C2ccccC2)C(F)(F)F)c(O)C1;
- OC1cc(OCC2ccc(C=C)cC2)ccC1-C1[nH]nc(C1-C1ccccC1)C(F)(F)F;
- OC1cc(OCC2c(F)cccC2Cl)ccC1-C1[nH]nc(C1-C1ccc(Cl)cC1)C(F)(F)F;
- OC1cc(OCC2ccc(cC2)[N+]([O-])=O)ccC1-C1[nH]nc(C1-C1ccc(Cl)cC1)C(F)(F)F;
- OC1cc(OCC2ccc(Br)cC2)ccC1-C1[nH]nc(C1-C1ccc(Cl)cC1)C(F)(F)F;
- COC1ccc(cC1)-C1c(n[nH]C1-C1ccc(OCC2ccc(F)cC2)cC1O)C(F)(F)F;
- COC1ccccC1-C1c(n[nH]C1-C1ccc(OCC(C)=C)cC1O)C(F)(F)F;
- COC1ccccC1-C1c(n[nH]C1-C1ccc(OCC2ccc(C)cC2)cC1O)C(F)(F)F;
- COC1ccccC1-C1c(n[nH]C1-C1ccc(OCC2ccccC2)cC1O)C(F)(F)F;
- OC1cc(OCC2ccc(cC2)[N+]([O-])=O)ccC1-C1[nH]nc(C1-C1ccccC1)C(F)(F)F;
- COC1ccc(cC1)-C1c(n[nH]C1-C1ccc(OCC2ccccC2)cC1O)C(F)(F)F;
- OC1ccc(-C2n[nH]c(C2-C2ccc(Br)cC2)C(F)(F)F)c(O)C1;
- COC1ccc(-C2n[nH]c(C)C2-C2ccc(Cl)cC2)c(O)C1;
- CCOC1ccc(-C2n[nH]c(C)C2-C2ccc(Cl)cC2)c(O)C1;
- COC1ccc(-C2n[nH]cC2-C2ccc(Cl)cC2)c(O)C1;
- NNC(=O)COC1ccc(-C2n[nH]c(C2-C2ccc(Cl)cC2)C(F)(F)F)c(O)C1;
- OC1cc(OCC2ccccC2Cl)ccC1-C1n[nH]c(C1-C1ccc(Cl)cC1)C(F)(F)F;
- COC1ccccC1-C1c(n[nH]C1C(F)(F)F)-C1ccc(OCC(=O)NN)cC1O;
- COC1ccc(cC1OC)-C1c(n[nH]C1-C1ccc(OCC2ccc(C)cC2)cC1O)C(F)(F)F;
- COC1ccc(cC1OC)-C1c(n[nH]C1-C1ccc(OCC2ccccC2)cC1O)C(F)(F)F;
- CC(=C)COC1ccc(-C2[nH]nc(C2-C2ccc(Cl)cC2)C(F)(F)F)c(O)C1;
- COC1ccccC1-C1c(n[nH]C1-C1ccc(OCC=C(C)C)cC1O)C(F)(F)F.

The compounds to be used as an antibacterial or antibiotic against a pathogen of the present invention can also be represented by their more conventional structural formula. In this embodiment, the compound of formula I is selected from the group consisting of: 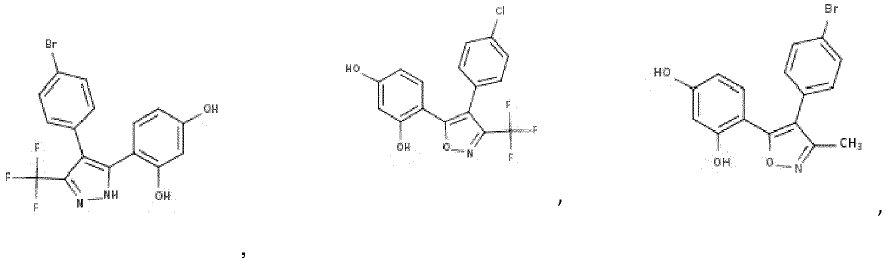 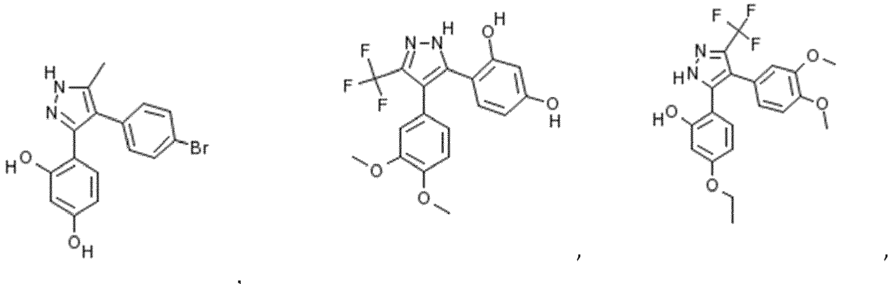 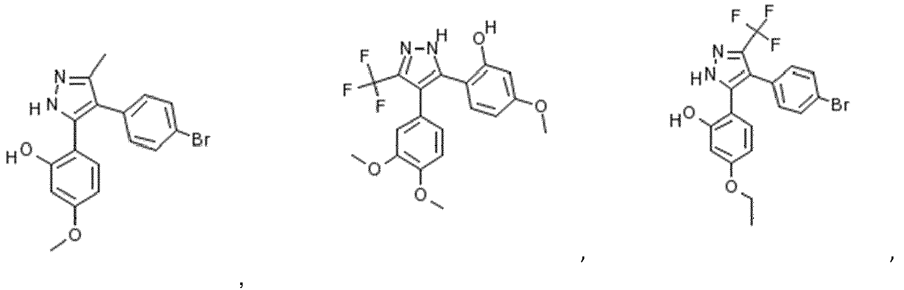 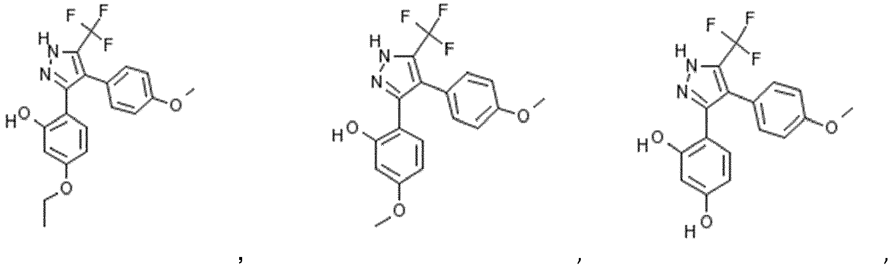 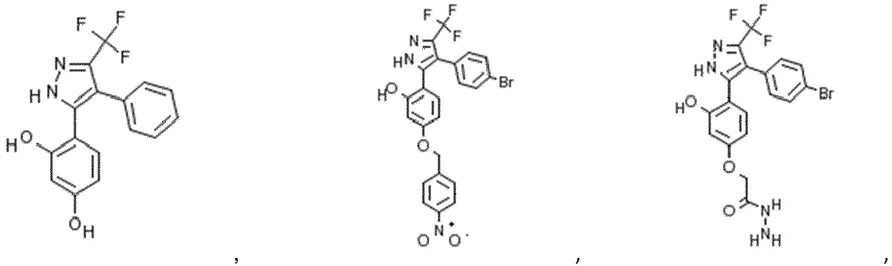 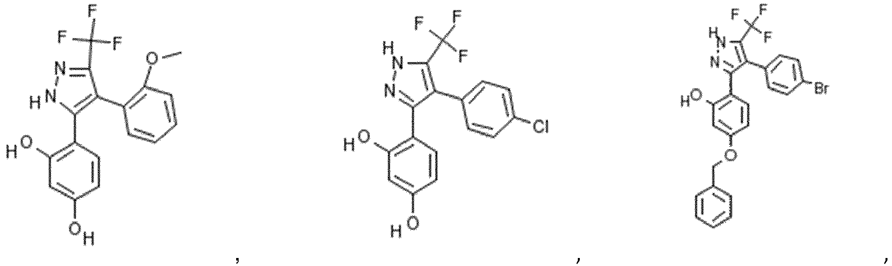 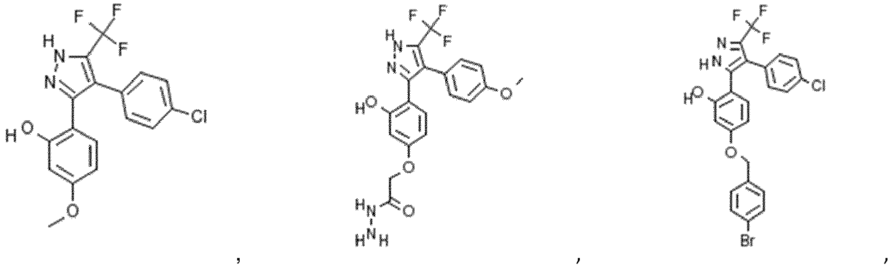 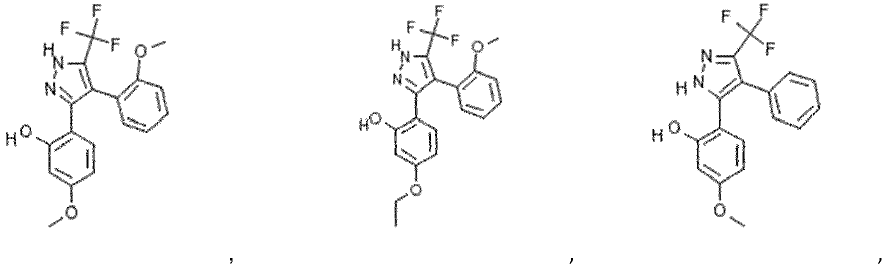 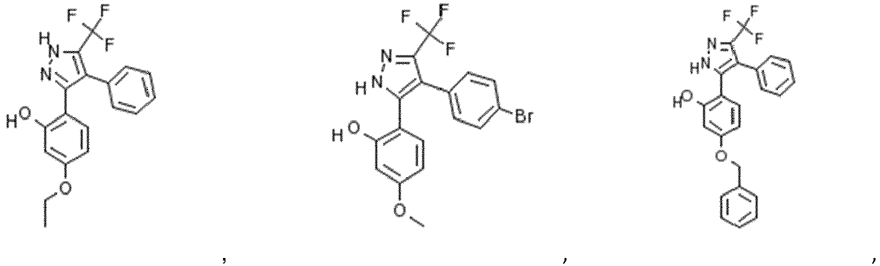 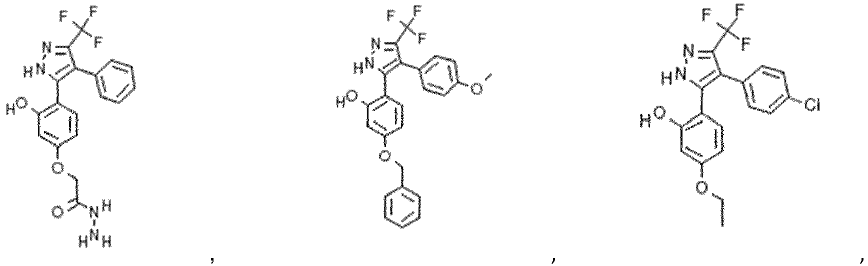 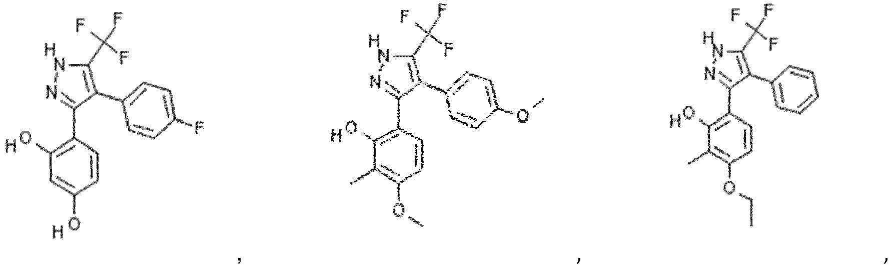 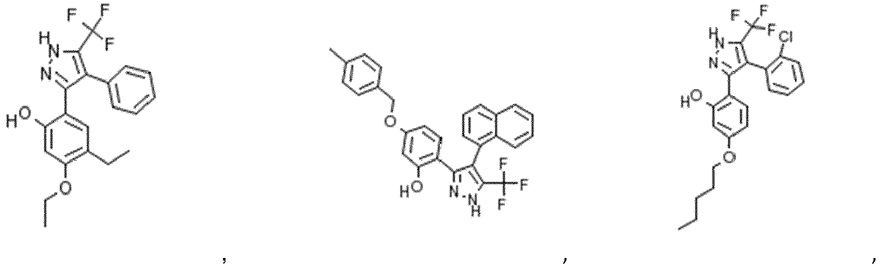 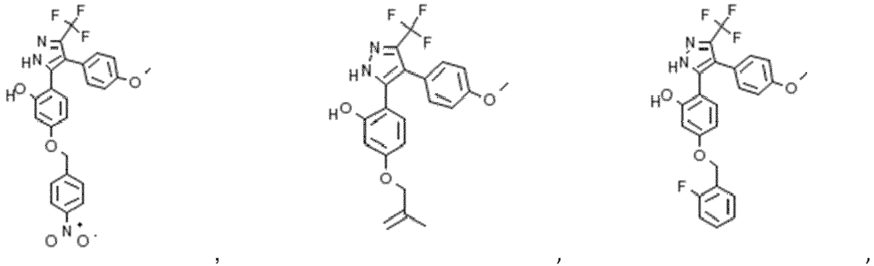 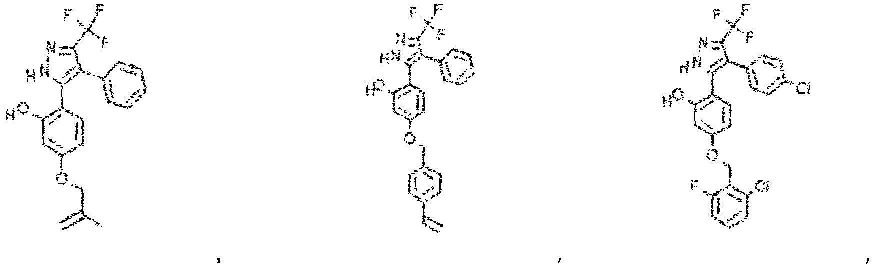 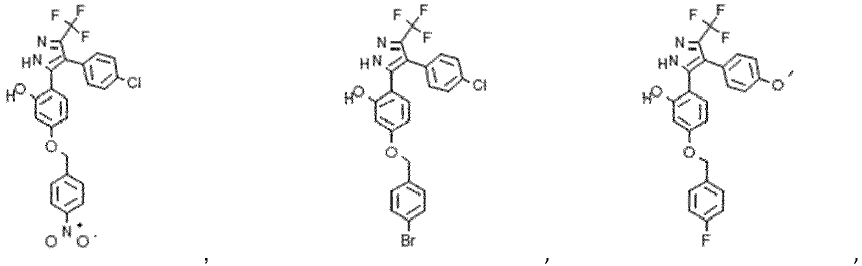 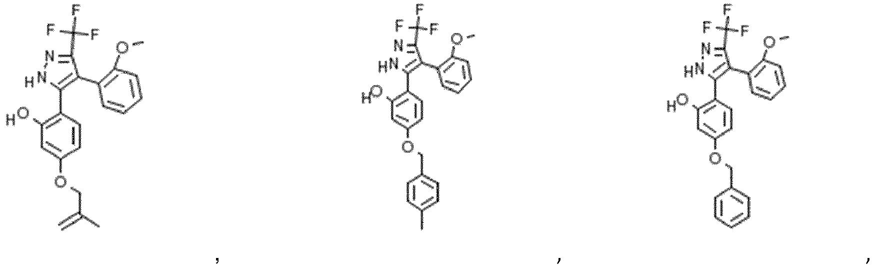 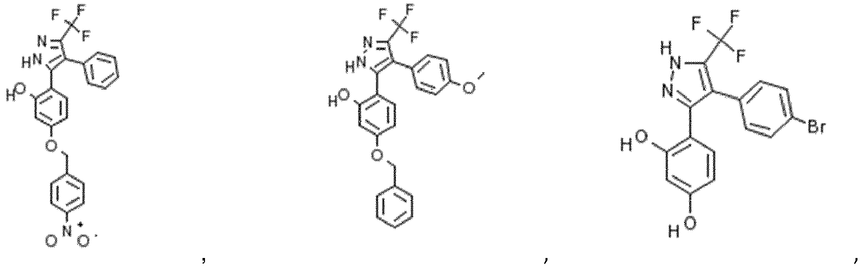 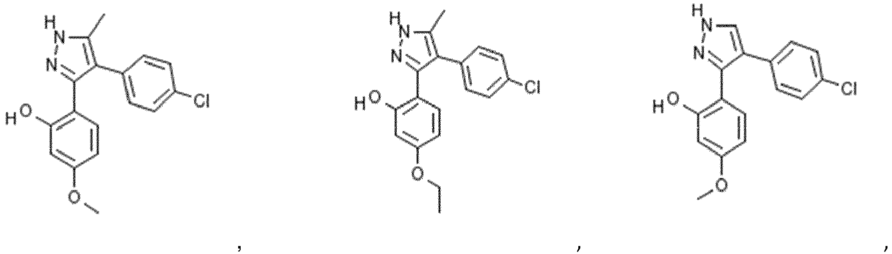 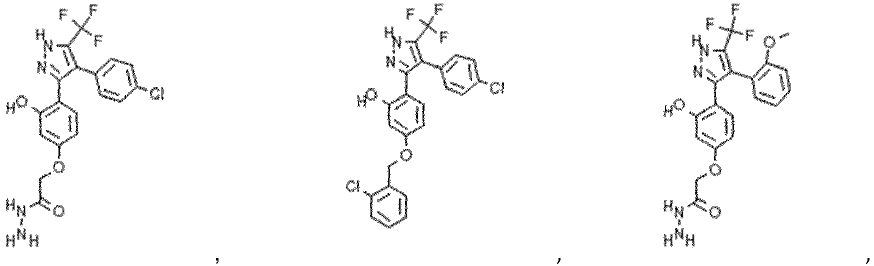 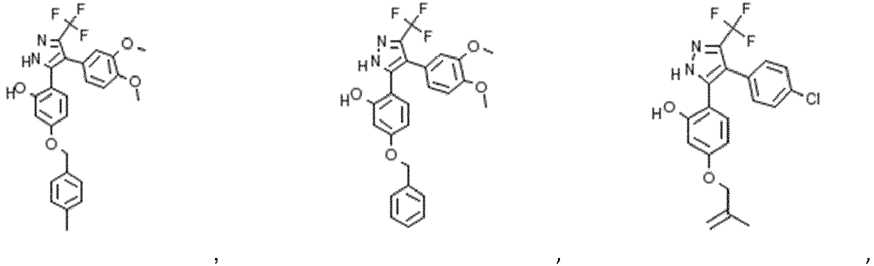 and

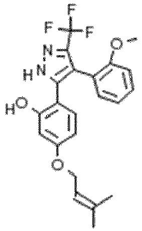

The compounds can be used as an antibacterial agent against gram-positive and/or gram-negative bacteria.

In a preferred embodiment of the invention, the compounds can be used as a medicament against a pathogen. The medicament can be an antibacterial agent and/or an antibiotic agent.

In certain embodiments, the pathogen is selected from the group consisting of *Staphylococcus, Escherichia, Streptococcus, Klebsiella, Mycobacterium, Pseudomonas, Clostridium, Acinetobacter, Enterococcus* and *Pasteurella Bacillus anthracis. Listeria monocytogenes, Corynebacterium diphtheriae, Salmonella, Shigella, Moraxella, Helicobacter, Legionella, Vibrio, Rickettsia, Neisseria, Actinobacter,* preferably *Staphylococcus, Escherichia, Streptococcus, Mycobacterium, Pseudomonas, Enterococcus* , and *Pasteurella* . In preferred embodiments, the pathogen is selected from the group consisting of *Staphylococcus aureus Newman, Streptococcus suis, Mycobacterium marinum, Pasteurella multocida, Escherichia coli,* and *Enterococcus faecium.*

In certain preferred embodiments, the pathogen is selected from the group consisting of *Staphylococcus aureus Newman, Streptococcus suis, Mycobacterium marinum, Pasteurella multocida, Escherichia coli,* and *Enterococcus faecium,* and the compound is selected from the group consisting of 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol, 2-[4-(4-bromophenyl)-5-(trifluoromethyl)-1∼{H}-pyrazol-3-yl]-5-(4-nitrobenzyl)oxy-phenol, 2-[4-[4-(4-bromophenyl)-5-(trifluoromethyl)-1∼{H}-pyrazol-3-yl]-3-hydroxy-phenoxy]acetohydrazide, 4-[4-(4-chlorophenyl)-5-(trifluoromethyl)-1∼{H}-pyrazol-3-yl]resorcinol, 2-[3-hydroxy-4-[4-(4-methoxyphenyl)-5-(trifluoromethyl)-1∼{H}-pyrazol-3-yl]phenoxy]acetohydrazide, 5-(4-bromobenzyl)oxy-2-[4-(4-chlorophenyl)-5-(trifluoromethyl)-1∼{H}-pyrazol-3-yl]phenol, 5-ethoxy-2-[4-(2-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 5-benzoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 5-benzoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 3-methoxy-6-[4-(4-methoxyphenyl)-5-(trifluoromethyl)-1∼{H}-pyrazol-3-yl]-2-methyl-phenol, 5-(2-chloro-6-fluorobenzyl)oxy-2-[4-(4-chlorophenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-(4-nitrobenzyl)oxy-phenol, 5-(4-bromobenzyl)oxy-2-[4-(4-chlorophenyl)-5-(trifluoromethyl)-1∼{H}-pyrazol-3-yl]phenol, 2-[4-(4-chlorophenyl)-1∼{H}-pyrazol-5-yl]-5-methoxy-phenol, 5-(2-chlorobenzyl)oxy-2-[4-(4-chlorophenyl)-5-(trifluoromethyl)-1∼{H}-pyrazol-3-yl]pheno, 2-[3-hydroxy-4-[4-(2-methoxyphenyl)-5-(trifluoromethyl)-1∼{H}-pyrazol-3-yl]phenoxy]acetohydrazide, 2-[4-(2-methoxyphenyl)-5-(trifluoromethyl)-1∼{H}-pyrazol-3-yl]-5-(3-methylbut-2-enoxy)phenol, 5-(4-methylbenzyl)oxy-2-[4-(1-naphthyl)-5-(trifluoromethyl)-1∼{H}-pyrazol-3-yl]phenol, 5-(2-fluorobenzyl)oxy-2-[4-(4-methoxyphenyl)-5-(trifluoromethyl)-1∼{H}-pyrazol-3-yl]phenol, 5-(2-methylallyloxy)-2-[4-phenyl-5-(trifluoromethyl)-1∼{H}-pyrazol-3-yl]phenol, preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-(4-bromobenzyl)oxy-2-[4-(4-chlorophenyl)-5-(trifiuoromethyl)-1∼{H}-pyrazol-3-yl]phenol, 5-ethoxy-2-[4-(2-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 5-benzoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 5-(4-bromobenzyl)oxy-2-[4-(4-chlorophenyl)-5-(trifluoromethyl)-1∼{H}-pyrazol-3-yl]phenol, 2-[4-(4-chlorophenyl)-)-5-methyl-1∼{H}-pyrazol-3-yl]-5-ethoxy-phenol, 2-[4-(4-chlorophenyl)-1∼{H}-pyrazol-5-yl]-5-methoxy-phenol, 5-(2-chlorobenzyl)oxy-2-[4-(4-chlorophenyl)-5-(trifluoromethyl)-1∼{H}-pyrazol-3-yl]pheno, 2-[3-hydroxy-4-[4-(2-methoxyphenyl)-5-(trifluoromethyl)-1∼{H}-pyrazol-3-yl]phenoxy]acetohydrazide, 2-[4-(3,4-dimethoxyphenyl)-5-(trifluoromethyl)-1∼{H}-pyrazol-3-yl]-5-(4-methylbenzyl)oxy-phenol, 2-[4-(4-chlorophenyl)-5-(trifluoromethyl)-1∼{H}-pyrazol-3-yl]-5-(2-methylallyloxy)phenol, 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol, more preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-chlorophenyl)-3-(trifluoromethyl)isoxazol-5-yl]resorcinol, 4-[4-(4-bromophenyl)-3-methyl-isoxazol-5-yl]resorcinol. In certain preferred embodiments, the pathogen is selected from the group consisting of *Staphylococcus aureus Newman,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-(4-bromobenzyl)oxy-2-[4-(4-chlorophenyl)-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 5-benzoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol. In certain preferred embodiments, the pathogen is selected from the group consisting of *Streptococcus suis,* and the compound is selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 2-[4-(4-chlorophenyl)-5-methyl-1-{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol, preferably selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol.

In certain preferred embodiments, the pathogen is selected from the group consisting of *Pasteurella multocida,* and the compound is selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 2-[4-(4-chlorophenyl)-5-(trifluoromethyl)-1∼{H}-pyrazol-3-yl]-5-ethoxy-phenol, 2-[4-(4-chlorophenyl)-)-5-methyl-1∼{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol.

In certain preferred embodiments, the pathogen is selected from the group consisting of *Mycobacterium marinum,* and the compound is selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-methyl-1∼{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol, preferably 2-[4-(3, 4-dimethoxyphenyl)-5-(trifluoromethyl)-1∼{H}-pyrazol-3-yl]-5-ethoxy-phenol.

In certain preferred embodiments, the pathogen is selected from the group consisting of *Escherichia coli,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-ethoxy-2-[4-(2-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol, preferably selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-chlorophenyl)-3-(trifluoromethyl)isoxazol-5-yl]resorcinol, 4-[4-(4-bromophenyl)-3-methyl-isoxazol-5-yl]resorcinol.

In certain preferred embodiments, the pathogen is selected from the group consisting of *Enterococcus faecium,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 2-[4-(4-chlorophenyl)-5-methyl-1∼{H}-pyrazol-3-yl]-5-ethoxy-phenol, 2-[4-(4-chlorophenyl)-1∼{H}-pyrazol-5-yl]-5-methoxy-phenol, 5-(2-chlorobenzyl)oxy-2-[4-(4-chlorophenyl)-5-(trifluoromethyl)-1∼{H}-pyrazol-3-yl]pheno, 2-[3-hydroxy-4-[4-(2-methoxyphenyl)-5-(trifluoromethyl)-1∼{H}-pyrazol-3-yl]phenoxy]acetohydrazide, 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 4-[4-(4-bromophenyl)-3-methyl-isoxazol-5-yl]resorcinol.

The compounds of the invention express a selectivity index of more than 3.

In preferred embodiments the compounds of the invention express a selectivity index of more than 5.

In more preferred embodiments the compounds of the invention express a selectivity index of more than of more than 15 (see Table 4-6).

It will be clear that the compounds can be used in combination without listing each and every permutation of these specific compounds separately or with other compounds that are within the scope of the invention as defined in the appended claims.

In a further aspect the invention pertains to the use of the compounds of the invention in a pharmaceutical composition. To that end, the compounds are preferably formulated using general galenic principles.

### Examples

### Compounds

Compounds were commercially available from Molport (www.molport.com) and were used as received.

### Method for determining minimal inhibitor concentration (MIC) and minimal bactericidal concentration (MIB).

In short, a stock solution of 5 mg/mL of HKIs in DMSO was made. This stock was diluted to 50 µg/mL in broth in a 96 well plate. Dilutions were made (factor 2) directly in the plate with a multi-channel pipet. Bacteria cultured in suspension overnight were then added to the plate (except negative control) and the plate was incubated for 24 hours. After 24 hours the OD600 was measured using a spectrophotometer. A positive control (with bacteria, without HKI) was also measured on each plate. The MICs were not determined in duplo/triplicate in the same experiment, however, the MICs of several HKIs were measured in different experiments and no major differences (max. 1x dilution) were found in the MICs between the experiments.

Bacterial strains used in this study for antibacterial susceptibility testing (Table S2) were propagated using standard microbiological procedures. Minimal inhibitory concentrations (MICs) were determined following a standard double-dilution method (42), 73. MICs were recorded as the lowest concentration of the compound where no visible growth was observed. After plating the dilutions around the MIC or growing them in fresh MH media, minimal bactericidal concentration (MBC) was recorded as the lowest concentration of the compound at which no colonies were formed or no growth was observed, respectively. For M. *marinum,* MICs were determined in Middlebrook 7H9 broth (Difco) and MICs were determined after 3 days incubation at 30 °C.

Grow the bacterial colonies to be tested from single colonies on an agar plate in LB broth or suitable bacterial growth medium overnight at 37°C. Prepare a 5 mg/mL stock solution of compounds to be tested in DMSO. One 96-well plate per bacterial strain was prepared. First, put 50 uL of medium to each well. Add 48 µL of medium to all wells in row A. Pipet 2 µL of antimicrobial master stock into the top well (row A). Beware, the antimicrobial concentration will be diluted 2x after adding the bacterial culture. Total volume in each well will be 100 µL. In A11, pipet 2 µL of DMSO. Make a serial dilution from row A to row H, pipetting 50 µL from the previous row into the new one. Mix well by pipetting up and down. Discard the last 50 µL so that there is 50 µl in each well In a clean, new plastic pipetting cup, dilute the overnight bacterial culture 100 times. Add 100 µL of bacterial overnight culture to 9900 µL of medium. Mix the suspension well. Once the serial dilutions are complete for all antimicrobial compounds, add 50 µL of diluted overnight bacterial culture to each well (except column 12). Mix by pipetting or put the plate on the shaker. Per 2 plates, close with parafilm around the edges and place in the incubator overnight. After overnight culturing, measure bacterial growth on the SpectraMax. The lowest concentration where no growth is observed is the MIC. To determine the MIB pipette 5 µL from each well of the 96 well plate where now growth was observed on to an LB agar plate, without antimicrobial. Put the plates in the incubator to grow overnight. The minimal concentration where no growth can be seen on the agar plate is the MBC.

### Cell toxicity

Toxicity was primarily determined by measuring cytotoxicity with the Alamar blue assay. In short, cells (HEK293, NRK-52E and Caco-2) were plated in 96-well plates and cultured overnight. HKI compounds were diluted to concentrations ranging from 0.39 to 500 µg/mL in serum-free medium and added to the cells.

After exposure, cells were incubated for 24 hours. Experiments were mostly performed by single measurement. Four compounds were measured in triplicate in HEK293 and Caco-2 cells which resulted in appropriate relative standard deviations (approx. 10%) for biological assays. After 24 hours HKIs were removed from the cells and Alamar blue was added, incubated for 45 minutes and fluorescence was measured on a spectrophotometer. Alamar blue measures the conversion of blue, low fluorescent resazurin into high fluorescent pink resorufin. This conversion can only be made in viable mitochondria in viable cells and therefore this dye provides a measure for cytotoxicity. The fluorescence results were translated from the spectrophotometer to inhibition curves showing cell viability (against a vehicle control of 1% DMSO) in Graphpad Prism and IC50 values were determined (in Prism) from these curves.

The results are presented in Table 4-6

### References:

(1) J. O'Neill, Tackling drug-resistant infections globally: final report and recommendations, Rev. Antimicrob. Resist. (2016) 84.
(2) Front Microbiol. 2019; 10: 539. Published online 2019 Apr 1. doi: 10.3389/fmicb.2019.00539 PMCID: PMC6452778 PMID: 30988669 Emerging Strategies to Combat ESKAPE Pathogens in the Era of Antimicrobial Resistance: A Review Mansura S. Mulani, Ekta E. Kamble, Shital N. Kumkar, Madhumita S. Tawre, and Karishma R. Pardesi
(3) Bacterial histidine kinases as novel antibacterial drug targets. Bem AE, Velikova N, Pellicer MT, Baarlen Pv, Marina A, Wells JM. ACS Chem Biol. 2015 Jan 16;10(1):213-24. doi: 10.1021/cb5007135. Epub 2014 Dec 26. Review.
(4) WalK, the Path towards New Antibacterials with Low Potential for Resistance Development. Velikova N, Bem AE, van Baarlen P, Wells JM, Marina A. ACS Med Chem Lett. 2013 Sep 18;4(10):891-4. doi: 10.1021/ml400320s. eCollection 2013 Oct 10.
(5) Putative histidine kinase inhibitors with antibacterial effect against multi-drug resistant clinical isolates identified by in vitro and in silico screens. Velikova N, Fulle S, Manso AS, Mechkarska M, Finn P, Conlon JM, Oggioni MR, Wells JM, Marina A. Sci Rep. 2016 May 13;6:26085. doi: 10.1038/srep26085.
(6) Broadening the antibacterial spectrum of histidine kinase autophosphorylation inhibitors via the use of ε-poly-L-lysine capped mesoporous silica-based nanoparticles. Velikova N, Mas N, Miguel-Romero L, Polo L, Stolte E, Zaccaria E, Cao R, Taverne N, Murguía JR, Martinez-Manez R, Marina A, Wells J. Nanomedicine. 2017 Feb;13(2):569-581. doi: 10.1016/j.nano.2016.09.011. Epub 2016 Oct 5.
(7) ioorg Med Chem Lett. 2017 Dec 1;27(23):5235-5244. doi: 10.1016/j.bmcl.2017.10.036. Epub 2017 Oct 19. Repurposing Hsp90 inhibitors as antibiotics targeting histidine kinases. Vo CD, Shebert HL Zikovich S, Dryer RA, Huang TP, Moran LJ, Cho J, Wassarman DR, Falahee BE1, Young PD, Gu GH, Heinl JF, Hammond JW, Jackvony TN, Frederick TE, Blair JA.

**Table 1: Compounds of the invention**

| **#** | **Name** | **Structure** | **SMILES Representation** | **Systematic name** |
|---|---|---|---|---|
| **1.** | **AND-1** | 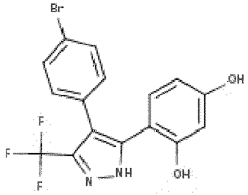 | OC₁ccc(-C₂[nH]nc(C₂-C₂ccc(Br)cC₂)C(F)(F)F)c(O) C₁ | 4-[4-(4-bromophenyl)-5-(trifluoromethyl)-1∼{H}-pyrazol-3-yl]resorcinol |
| **2.** | **AND-2** | 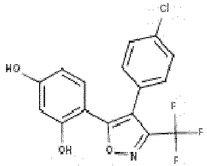 | OC₁ccc(-C₂onc(C₂-C₂ccc(Cl)cC₂)C(F)(F)F)c(O) C₁ | 4-[4-(4-chlorophenyl)-3-(trifluoromethyl)isox azol-5-yl]resorcinol |
| **3.** | **AND-3** | 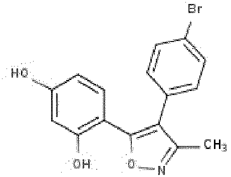 | CC₁noc(C₁-C₁ccc(Br)cC₁)-C₁ccc(O)cC₁O | 4-[4-(4-bromophenyl)-3-methyl-isoxazol-5-yl]resorcinol |
| **4.** | **May-1** | 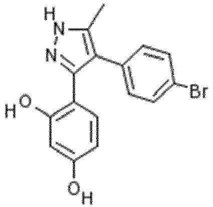 | CC₁[nH]nc(C₁-C₁ccc(Br)cC₁)-C₁ccc(O)cC₁O | 4-[4-(4-bromophenyl)-5-methyl-1∼{H}-pyrazol-3-yl]resorcinol |
| **5.** | **May-2** | 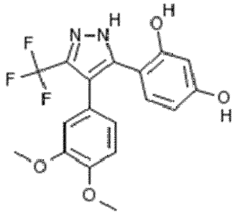 | COC₁ccc(cC₁OC)-C₁c([nH]nC₁C(F)(F)F)-C₁ccc(O)cC₁O | 4-[4-(3,4-dimethoxyphenyl)-5-(trifluoromethyl)-1∼{H}-pyrazol-3-yl]resorcinol |
| **6.** | **May-3** | 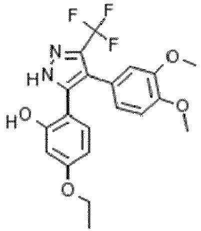 | CCOC₁ccc(-C₂[nH]nc(C₂-C₂ccc(OC)c(OC)C₂)C(F)(F)F )c(O)C₁ | 2-[4-(3, 4-dimethoxyphenyl)-5-(trifluoromethyl)-1∼{H}-pyrazol-3-yl]-5-ethoxy-phenol |
| **7.** | **May-4** | 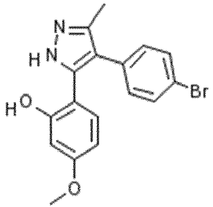 | COC₁ccc(-C₂[nH]nc(C)C₂-C₂ccc(Br)cC₂)c(O)C₁ | 2-[4-(4-bromophenyl)-5-methyl-1∼{H}-pyrazol-3-yl]-5-methoxy-phenol |
| **8.** | **May-5** | 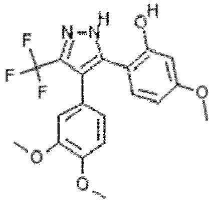 | COC₁ccc(-C₂[nH]nc(C₂-C₂ccc(OC)c(OC)C₂)C(F)(F)F )c(O)C₁ | 2-[4-(3, 4-dimethoxyphenyl)-5-(trifluoromethyl)-1∼{H}-pyrazol-3-yl]-5-methoxy-phenol |
| **9.** | **May-6** | 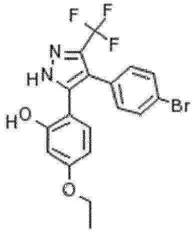 | CCOC₁ccc(-C₂[nH]nc(C₂-C₂ccc(Br)cC₂)C(F)(F)F)c(O) C₁ | 2-[4-(4-bromophenyl)-5-(trifluoromethyl)-1∼{H}-pyrazol-3-yl]-5-ethoxy-phenol |
| **10.** | **May-7** | 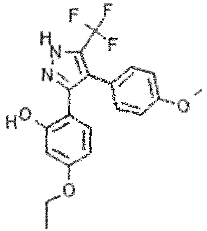 | CCOC₁ccc(-C₂n[nH]c(C₂-C₂ccc(OC)cC₂)C(F)(F)F)c(O )C₁ | 5-ethoxy-2-[4-(4-methoxyphenyl)-5-(trifluoromethyl)-1∼{H}-pyrazol-3-yl]phenol |
| **11.** | **May-8** | 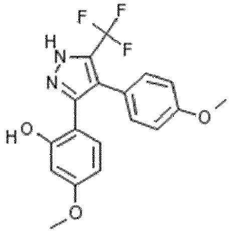 | COC₁ccc(cC₁)-C₁c(n[nH]C₁C(F)(F)F)-C₁ccc(OC)cC₁O | 5-ethoxy-2-[4-(4-methoxyphenyl)-5-(trifluoromethyl)-1∼{H}-pyrazol-3-yl]phenol |
| **12.** | **May-9** | 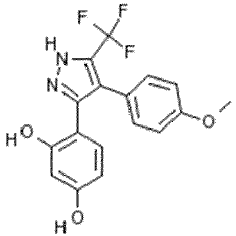 | COC₁ccc(cC₁)-C₁c(n[nH]C₁C(F)(F)F)-C₁ccc(O)cC₁O | 4-[4-(4-methoxyphenyl)-5-(trifluoromethyl)-1∼{H}-pyrazol-3-yl]resorcinol |
| **13.** | **May-10** | 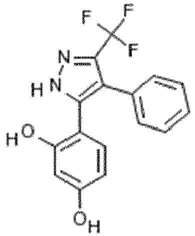 | OC₁ccc(-C₂[nH]nc(C₂-C₂ccccC₂)C(F)(F)F)c(O)C₁ | 4-[4-phenyl-5-(trifluoromethyl)-1∼{H}-pyrazol-3-yl]resorcinol |
| **14.** | **May-11** | 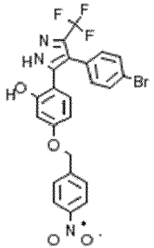 | OC₁cc(OCC₂ccc(cC₂)[N+]([O -])=O)ccC₁-C₁[nH]nc(C₁-C₁ccc(Br)cC₁)C(F)(F)F | 2-[4-(4-bromophenyl)-5-(trifluoromethyl)-1∼{H}-pyrazol-3-yl]-5-(4-nitrobenzyl)oxyphenol |
| **15.** | **May-12** | 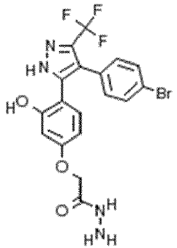 | NNC(=O)COC₁ccc(-C₂[nH]nc(C₂-C₂ccc(Br)cC₂)C(F)(F)F)c(O) C₁ | 2-[4-[4-(4-bromophenyl)-5-(trifluoromethyl)-1∼{H}-pyrazol-3-yl]-3-hydroxy-phenoxy]acetohydrazide |
| **16.** | **May-13** | 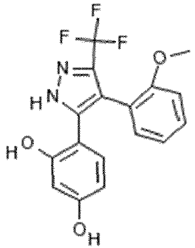 | COC₁ccccC₁-C₁c([nH]nC₁C(F)(F)F)-C₁ccc(O)cC₁O | 4-[4-(2-methoxyphenyl)-5-(trifluoromethyl)-1∼{H}-pyrazol-3-yl]resorcinol |
| **17.** | **May-14** | 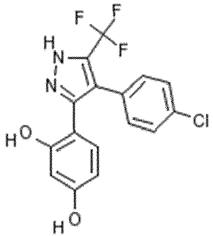 | OC₁ccc(-C₂n[nH]c(C₂-C₂ccc(Cl)cC₂)C(F)(F)F)c(O) C₁ | 4-[4-(4-chlorophenyl)-5-(trifluoromethyl)-1∼{H}-pyrazol-3-yl]resorcinol |
| **18.** | **May-15** | 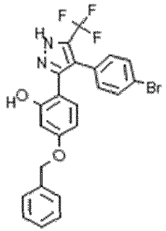 | OC₁cc(OCC₂ccccC₂)ccC₁-C₁n[nH]c(C₁-C₁ccc(Br)cC₁)C(F)(F)F | 5-benzoxy-2-[4-(4-bromophenyl)-5-(trifluoromethyl)-1∼{H}-pyrazol-3-yl]phenol |
| **19.** | **May-16** | 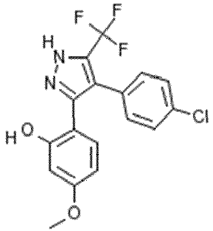 | COC₁ccc(-C₂n[nH]c(C₂-C₂ccc(Cl)cC₂)C(F)(F)F)c(O) C₁ | 2-[4-(4-chlorophenyl)-5-(trifluoromethyl)-1∼{H}-pyrazol-3-yl]-5-methoxy-phenol |
| **20.** | **May-17** | 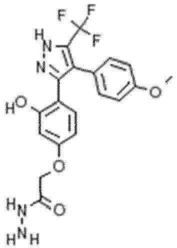 | COC₁ccc(cC1)-C₁c(n[nH]C₁C(F)(F)F)-C₁ccc(OCC(=O)NN)cC₁O | 2-[3-hydroxy-4-[4-(4-methoxyphenyl)-5-(trifluoromethyl)-1∼{H}-pyrazol-3-yl]phenoxy]acetohy drazide |
| **21.** | **May-18** | 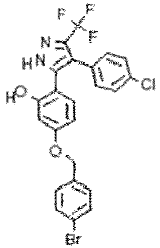 | OC₁cc(OCC₂ccc(Br)cC₂)ccC ₁-C₁[nH]nc(C₁-C₁ccc(Cl)cC₁)C(F)(F)F | 5-(4-bromobenzyl)oxy-2-[4-(4-chlorophenyl)-5-(trifluoromethyl)-1∼{H}-pyrazol-3-yl]phenol |
| **22.** | **May-19** | 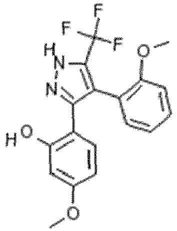 | COC₁ccc(-C₂n[nH]c(C₂-C₂ccccC₂OC)C(F)(F)F)c(O) C₁ | 5-methoxy-2-[4-(2-methoxyphenyl)-5-(trifluoromethyl)-1∼{H}-pyrazol-3-yl]phenol |
| **23.** | **May-20** | 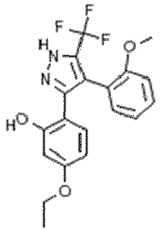 | CCOC₁ccc(-C₂n[nH]c(C₂-C₂ccccC₂OC)C(F)(F)F)c(O) C₁ | 5-ethoxy-2-[4-(2-methoxyphenyl)-5-(trifluoromethyl)-1∼{H}-pyrazol-3-yl]phenol |
| **24.** | **May-21** | 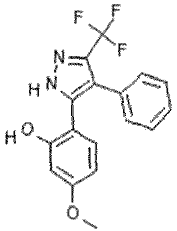 | COC₁ccc(-C₂[nH]nc(C₂-C₂ccccC₂)C(F)(F)F)c(O)C₁ | 5-methoxy-2-[4-phenyl-5-(trifluoromethyl)-1∼{H}-pyrazol-3-yl]phenol |
| **25.** | **May-22** | 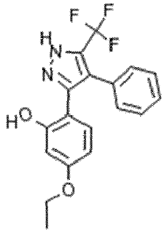 | CCOC₁ccc(-C₂n[nH]c(C₂-C₂ccccC₂)C(F)(F)F)c(O)C₁ | 5-ethoxy-2-[4-phenyl-5-(trifluoromethyl)-1∼{H}-pyrazol-3-yl]phenol |
| **26.** | **May-23** | 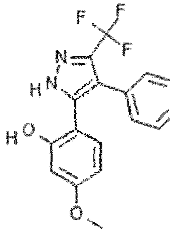 | COC₁ccc(-C₂[nH]nc(C₂-C₂ccc(Br)cC₂)C(F)(F)F)c(O) C₁ | 2-[4-(4-bromophenyl)-5-(trifluoromethyl)-1∼{H}-pyrazol-3-yl]-5-methoxy-phenol |
| **27.** | **May-24** | 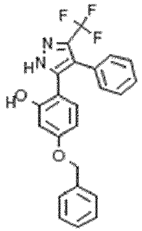 | OC₁cc(OCC₂ccccC₂)ccC₁-C₁[nH]nc(C₁-C₁ccccC₁)C(F)(F)F | 5-benzoxy-2-[4-phenyl-5-(trifluoromethyl)-1∼{H}-pyrazol-3-yl]phenol |
| **28.** | **May-25** | 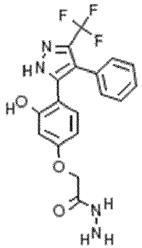 | NNC(=O)COC₁ccc(-C₂[nH]nc(C₂-C₂ccccC₂)C(F)(F)F)c(O)C₁ | 2-[3-hydroxy-4-[4-phenyl-5-(trifluoromethyl)-1∼{H}-pyrazol-3-yl]phenoxy]acetohy drazide |
| **29.** | **May-26** | 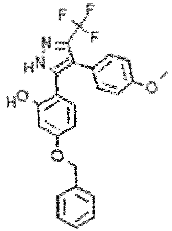 | COC₁ccc(cC₁)-C₁c(n[nH]C₁-C₁ccc(OCC₂ccccC₂)cC₁O)C( F)(F)F | 5-benzoxy-2-[4-(4-methoxyphenyl)-5-(trifluoromethyl)-1∼{H}-pyrazol-3-yl]phenol |
| **30.** | **May-27** | 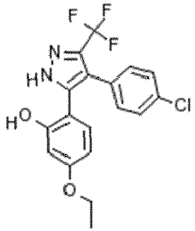 | CCOC₁ccc(-C₂[nH]nc(C₂-C₂ccc(Cl)cC₂)C(F)(F)F)c(O) C₁ | 2-[4-(4-chlorophenyl)-5-(trifluoromethyl)-1∼{H}-pyrazol-3-yl]-5-ethoxy-phenol |
| **31.** | **May-28** | 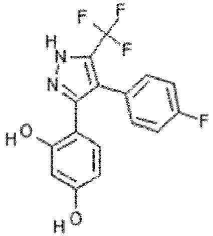 | OC₁ccc(-C₂n[nH]c(C₂-C₂ccc(F)cC₂)C(F)(F)F)c(O)C ₁ | 4-[4-(4-fluorophenyl)-5-(trifluoromethyl)-1∼{H}-pyrazol-3-yl]resorcinol |
| **32.** | **Sep-1** | 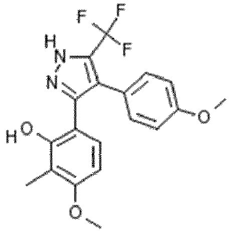 | COC₁ccc(cC₁)-C₁c(n[nH]C₁C(F)(F)F)-C₁ccc(OC)c(C)C₁O | 3-methoxy-6-[4-(4-methoxyphenyl)-5-(trifluoromethyl)-1∼{H}-pyrazol-3-yl]-2-methyl-phenol |
| **33.** | **Sep-2** | 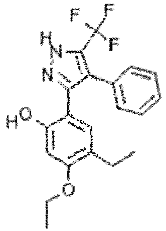 | CCOC₁cc(O)c(cC₁CC)-C₁n[nH]c(C₁-C₁ccccC₁)C(F)(F)F | 5-ethoxy-4-ethyl-2-[4-phenyl-5-(trifluoromethyl)-1∼{H}-pyrazol-3-yl]phenol |
| **34.** | **Sep-3** | 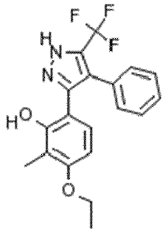 | CCOC₁ccc(-C₂n[nH]c(C₂-C₂ccccC₂)C(F)(F)F)c(O)C₁C | 3-ethoxy-2-methyl-6-[4-phenyl-5-(trifluoromethyl)-1∼{H}-pyrazol-3-yl]phenol |
| **35.** | **Sep-4** | 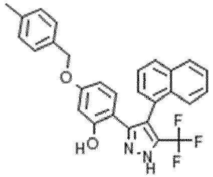 | CC₁ccc(COC₂ccc(-c3n[nH]c(c3-c3cccC₄ccccc34)C(F)(F)F)c( O)C₂)cC₁ | 5-(4-methylbenzyl)oxy-2-[4-(1-naphthyl)-5-(trifluoromethyl)-1∼{H}-pyrazol-3-yl]phenol |
| **36.** | **Sep-5** | 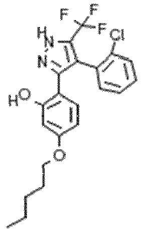 | CCCCCOC₁ccc(-C₂n[nH]c(C₂-C₂ccccC₂Cl)C(F)(F)F)c(O)C₁ | 2-[4-(2-chlorophenyl)-5-(trifluoromethyl)-1∼{H}-pyrazol-3-yl]-5-pentoxyphenol |
| **37.** | **Sep-6** | 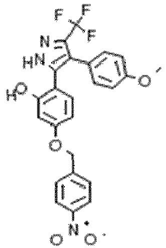 | COC₁ccc(cC₁)-C₁c(n[nH]C₁-C₁ccc(OCC₂ccc(cC₂)[N+]([O -])=O)cC₁O)C(F)(F)F | 2-[4-(4-methoxyphenyl)-5-(trifluoromethyl)-1∼{H}-pyrazol-3-yl]-5-(4-nitrobenzyl)oxyphenol |
| **38.** | **Sep-7** | 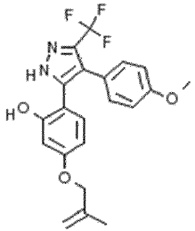 | COC₁ccc(cC₁)-C₁c(n[nH]C₁-C₁ccc(OCC(C)=C)cC₁O)C(F )(F)F | 2-[4-(4-methoxyphenyl)-5-(trifluoromethyl)-1∼{H}-pyrazol-3-yl]-5-(2-methylallyloxy)phen ol |
| **39.** | **Sep-8** | 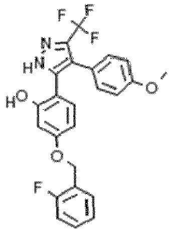 | COC₁ccc(cC₁)-C₁c(n[nH]C₁-C₁ccc(OCC₂ccccC₂F)cC₁O) C(F)(F)F | 5-(2-fluorobenzyl)oxy-2-[4-(4-methoxyphenyl)-5-(trifluoromethyl)-1∼{H}-pyrazol-3-yl]phenol |
| **40.** | **Sep-9** | 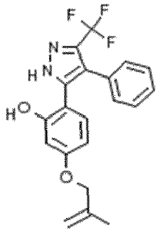 | CC(=C)COC₁ccc(-C₂[nH]nC(C₂-C₂ccccC₂)C(F)(F)F)c(O)C₁ | 5-(2-methylallyloxy)-2-[4-phenyl-5-(trifluoromethyl)-1∼{H}-py yl]phenol |
| **41.** | **Sep-10** | 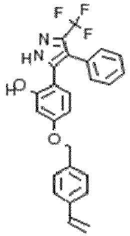 | OC₁cc(OCC₂ccc(C=C)cC₂)c cC₁-C₁[nH]nc(C₁-C₁ccccC₁)C(F)(F)F | 2-[4-phenyl-5-(trifluoromethyl)-1∼{H}-pyrazol-3-yl]-5-(4-vinylbenzyl)oxy-phenol |
| **42.** | **Sep-11** | 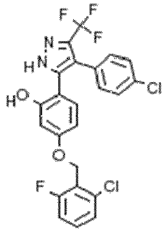 | OC₁cc(OCC₂c(F)cccC₂Cl)cc C₁-C₁[nH]nc(C₁-C₁ccc(Cl)cC₁)C(F)(F)F | 5-(2-chloro-6-fluorobenzyl)oxy-2-[4-(4-chlorophenyl)-5-(trifluoromethyl)-1∼{H}-pyrazol-3-yl]phenol |
| **43.** | **Sep-12** | 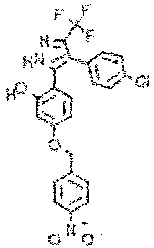 | OC₁cc(OCC₂ccc(cC₂)[N+]([O -])=O)ccC₁-C₁[nH]nc(C₁-C₁ccc(Cl)cC₁)C(F)(F)F | 2-[4-(4-chlorophenyl)-5-(trifluoromethyl)-1∼{H}-pyrazol-3-yl]-5-(4-nitrobenzyl)oxyphenol |
| **44.** | **Sep-13** | 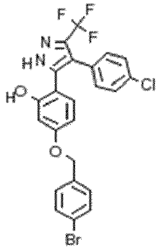 | OC₁cc(OCC₂ccc(Br)cC₂)ccC ₁-C₁[nH]nc(C₁-C₁ccc(Cl)cC₁)C(F)(F)F | 5-(4-bromobenzyl)oxy-2-[4-(4-chlorophenyl)-5-(trifluoromethyl)-1∼{H}-pyrazol-3-yl]phenol |
| **45.** | **Sep-14** | 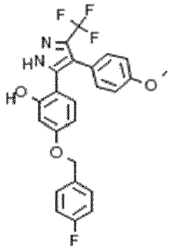 | COC₁ccc(cC₁)-C₁c(n[nH]C₁-C₁ccc(OCC₂ccc(F)cC₂)cC₁O )C(F)(F)F | 5-(4-fluorobenzyl)oxy-2-[4-(4-methoxyphenyl)-5-(trifluoromethyl)-1∼{H}-pyrazol-3-yl]phenol |
| **46.** | **Sep-15** | 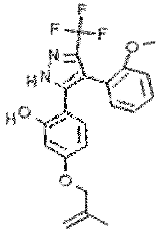 | COC₁ccccC₁-C₁c(n[nH]C₁-C₁ccc(OCC(C)=C)cC₁O)C(F )(F)F | 2-[4-(2-methoxyphenyl)-5-(trifluoromethyl)-1∼{H}-pyrazol-3-yl]-5-(2-methylallyloxy)phen ol |
| **47.** | Sep-16 | 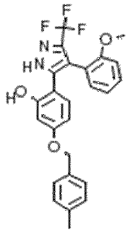 | COC₁ccccC₁-C₁c(n[nH]C₁-C₁ccc(OCC₂ccc(C)cC₂)cC₁O )C(F)(F)F | 2-[4-(2-methoxyphenyl)-5-(trifluoromethyl)-1∼{H}-pyrazol-3-yl]-5-(4-methylbenzyl)oxy-phenol |
| **48.** | **Sep-17** | 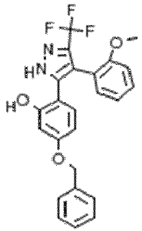 | COC₁ccccC₁-C₁c(n[nH]C₁-C₁ccc(OCC₂ccccC₂)cC₁O)C( F)(F)F | 5-benzoxy-2-[4-(2-methoxyphenyl)-5-(trifluoromethyl)-1∼{H}-pyrazol-3-yl]phenol |
| **49.** | **Sep-18** | 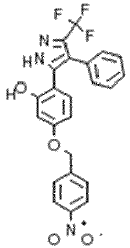 | OC₁cc(OCC₂ccc(cC₂)[N+]([O -])=O)ccC₁-C₁[nH]nc(C₁-C₁ccccC₁)C(F)(F)F | 5-(4-nitrobenzyl)oxy-2-[4-phenyl-5-(trifluoromethyl)-1∼{H}-pyrazol-3-yl]phenol |
| **50.** | **Sep-19** | 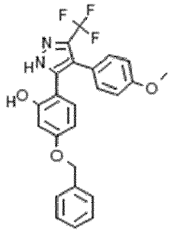 | COC₁ccc(cC₁)-C₁c(n[nH]C₁-C₁ccc(OCC₂ccccC₂)cC₁O)C( F)(F)F | 5-benzoxy-2-[4-(4-methoxyphenyl)-5-(trifluoromethyl)-1∼{H}-pyrazol-3-yl]phenol |
| **51.** | **Sep-20** | 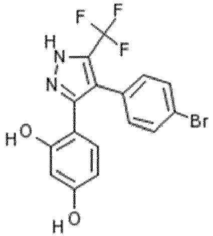 | OC₁ccc(-C₂n[nH]c(C₂-C₂ccc(Br)cC₂)C(F)(F)F)c(O) C₁4-[4-(4- | bromophenyl)-5-(trifluoromethyl)-1∼{H}-pyrazol-3-yl]resorcinol |
| **52.** | **Sep-21** | 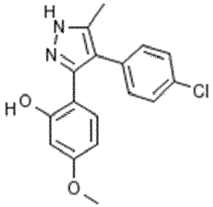 | COC₁ccc(-C₂n[nH]c(C)C₂-C₂ccc(Cl)cC₂)c(O)C₁2-[4-(4- | 2-[4-(4-chlorophenyl)-5-methyl-1∼{H}-pyrazol-3-yl]-5-methoxy-phenol |
| **53.** | **Sep-22** | 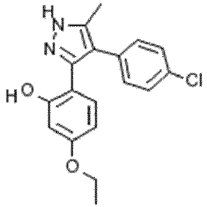 | CCOC₁ccc(-C₂n[nH]c(C)C₂-C₂ccc(Cl)cC₂)c(O)C₁2-[4-(4- | 2-[4-(4-chlorophenyl)--5-methyl-1∼{H}-pyrazol-3-yl]-5-ethoxy-phenol |
| **54**. | **Sep-23** | 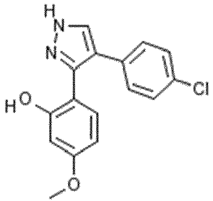 | COC₁ccc(-C₂n[nH]cC₂-C₂ccc(Cl)cC₂)c(O)C₁ | 2-[4-(4-chlorophenyl)-1∼{H}-pyrazol-5-yl]-5-methoxy-phenol |
| **55.** | **Sep-24** | 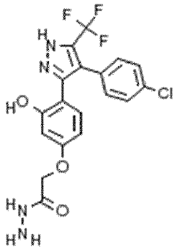 | NNC(=O)COC₁ccc(-C₂n[nH]c(C₂-C₂ccc(Cl)cC₂)C(F)(F)F)c(O) C₁ | 2-[4-[4-(4-chlorophenyl)-5-(trifluoromethyl)-1∼{H}-pyrazol-3-yl]-3-hydroxy-phenoxy]acetohydr azide |
| **56.** | **Sep-25** | 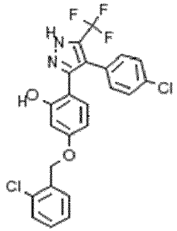 | OC₁cc(OCC₂ccccC₂C)ccC₁-C₁n[nH]c(C₁-C₁ccc(Cl)cC1)C(F)(F)F I | 5-(2-chlorobenzyl)oxy-2-[4-(4-chlorophenyl)-5-(trifluoromethyl)-1∼{H}-pyrazol-3-yl]pheno |
| **57.** | **Sep-26** | 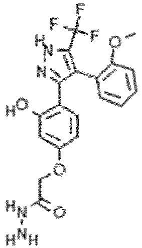 | COC₁ccccC₁-C₁c(n[nH]C₁C(F)(F)F)-C₁ccc(OCC(=O)NN)cC₁O | 2-[3-hydroxy-4-[4-(2-methoxyphenyl)-5-(trifluoromethyl)-1∼{H}-pyrazol-3-yl]phenoxy]acetohy drazide |
| **58.** | **Sep-27** | 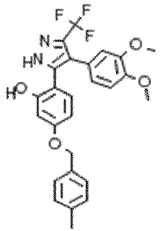 | COC₁ccc(cC₁OC)-C₁c(n[nH]C₁-C₁ccc(OCC₂ccc(C)cC₂)cC₁O )C(F)(F)F | 2-[4-(3,4-dimethoxyphenyl)-5-(trifluoromethyl)-1∼{H}-pyrazol-3-yl]-5-(4-methylbenzyl)oxy-phenol |
| **59.** | **Sep-28** | 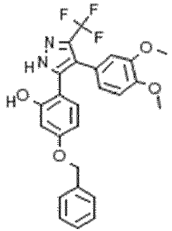 | COC₁ccc(cC₁OC)-C₁c(n[nH]C₁-C₁ccc(OCC₂ccccC₂)cC₁O)C( F)(F)F | 5-benzoxy-2-[4-(3, 4-dimethoxyphenyl)-5-(trifluoromethyl)-1∼{H}-pyrazol-3-yl]phenol |
| **60.** | **Sep-29** | 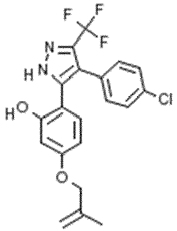 | CC(=C)COC₁ccc(-C₂[nH]nc(C₂-C₂ccc(Cl)cC₂)C(F)(F)F)c(O) C₁ | 2-[4-(4-chlorophenyl)-5-(trifluoromethyl)-1∼{H}-pyrazol-3-yl]-5-(2-methylallyloxy)phen ol |
| **61.** | **Sep-30** | 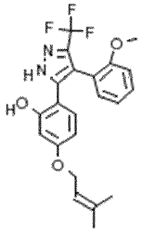 | COC₁ccccC₁-C₁c(n[nH]C₁-C₁ccc(OCC=C(C)C)cC₁O)C( F)(F)F | 2-[4-(2-methoxyphenyl)-5-(trifluoromethyl)-1∼{H}-pyrazol-3-yl]-5-(3-methylbut-2-enoxy)phenol |

**Table 2: substitution scheme**

| | | | | | | | | | | | | | |
|---|---|---|---|---|---|---|---|---|---|---|---|---|---|
| 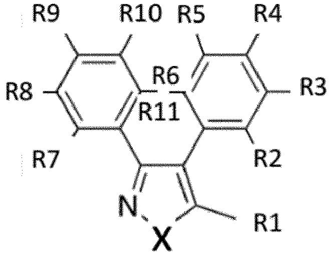 | | | | | | | | | | | | | |

| **#** | **Name** | **X** | **R1** | **R2** | **R3** | **R4** | **R5** | **R6** | **R7** | **R8** | **R9** | **R10** | **R11** |
|---|---|---|---|---|---|---|---|---|---|---|---|---|---|
| **1.** | **AND-1** | NH | CF₃ | H | H | Br | H | H | OH | H | OH | H | H |
| **2.** | **AND-2** | O | CF₃ | H | H | Cl | H | H | OH | H | OH | H | H |
| **3.** | **AND-3** | O | CH₃ | H | H | Br | H | H | OH | H | OH | H | H |
| **4.** | **May-1** | NH | CH₃ | H | H | Br | H | H | OH | H | OH | H | H |
| **5.** | **May-2** | NH | CF₃ | H | OCH₃ | OCH₃ | H | H | OH | H | OH | H | H |
| **6.** | **May-3** | NH | CF₃ | H | OCH₃ | OCH₃ | H | H | OH | H | OCH₂CH₃ | H | H |
| **7.** | **May-4** | NH | CH₃ | H | H | Br | H | H | OH | H | OCH₃ | H | H |
| **8.** | **May-5** | NH | CF₃ | H | OCH₃ | OCH₃ | H | H | OH | H | OCH₃ | H | H |
| **9.** | **May-6** | NH | CF₃ | H | H | Br | H | H | OH | H | OCH₂CH₃ | H | H |
| **10.** | **May-7** | NH | CF₃ | H | H | OCH₃ | H | H | OH | H | OCH₂CH₃ | H | H |
| **11.** | **May-8** | NH | CF₃ | H | H | OCH₃ | H | H | OH | H | OCH₃ | H | H |
| **12.** | **May-9** | NH | CF₃ | H | H | OCH₃ | H | H | OH | H | OH | H | H |
| **13.** | **May-0** | NH | CF₃ | H | H | H | H | H | OH | H | OH | H | H |
| **14.** | **May-11** | NH | CF₃ | H | H | Br | H | H | OH | H | OCH₂Ph-4-NO₂ | H | H |
| **15.** | **May-12** | NH | CF₃ | H | H | Br | H | H | OH | H | OCH₂(CO)NHNH₂ | H | H |
| **16.** | **May-13** | NH | CF₃ | OCH₃ | H | H | H | H | OH | H | OH | H | H |
| **17.** | **May-14** | NH | CF₃ | H | H | Cl | H | H | OH | H | OH | H | H |
| **18.** | **May-15** | NH | CF₃ | H | H | Br | H | H | OH | H | OCH₂Ph | H | H |
| **19.** | **May-16** | NH | CF₃ | H | H | Cl | H | H | OH | H | OCH₃ | H | H |
| **20.** | **May-17** | NH | CF₃ | H | H | OCH₃ | H | H | OH | H | OCH₂(CO)NHNH₂ | H | H |
| **21.** | **May-18** | NH | CF₃ | H | H | Cl | H | H | OH | H | OCH₂Ph-4-Br | H | H |
| **22.** | **May-19** | NH | CF₃ | OCH₃ | H | H | H | H | OH | H | OCH₃ | H | H |
| **23.** | **May-20** | NH | CF₃ | OCH₃ | H | H | H | H | OH | H | OCH₂CH₃ | H | H |
| **24.** | **May-21** | NH | CF₃ | H | H | H | H | H | OH | H | OCH₃ | H | H |
| **25.** | **May-22** | NH | CF₃ | H | H | H | H | H | OH | H | OCH₂CH₃ | H | H |
| **26.** | **May-23** | NH | CF₃ | H | H | Br | H | H | OH | H | OCH₃ | H | H |
| **27.** | **May-24** | NH | CF₃ | H | H | H | H | H | OH | H | OCH₂Ph | H | H |
| **28.** | **May-25** | NH | CF₃ | H | H | H | H | H | OH | H | OCH₂(CO)NHNH₂ | H | H |
| **29.** | **May-26** | NH | CF₃ | H | H | OCH₃ | H | H | OH | H | OCH₂Ph | H | H |
| **30.** | **May-27** | NH | CF₃ | H | H | Cl | H | H | OH | H | OH | H | H |
| **31.** | **May-28** | NH | CF₃ | H | H | F | H | H | OH | H | OH | H | H |
| **32.** | **Sep-1** | NH | CF₃ | H | H | OCH₃ | H | H | OH | CH₃ | OCH₃ | H | H |
| **33.** | **Sep-2** | NH | CF₃ | H | H | H | H | H | OH | H | OCH₂CH₃ | CH₂CH₃ | H |
| **34.** | **Sep-3** | NH | CF₃ | H | H | H | H | H | OH | CH₃ | OCH₂CH₃ | H | H |
| **35.** | **Sep-4** | NH | CF₃ | 1-naftyl | | | | | OH | H | OCH₂Ph-4-CH₃ | H | H |
| **36.** | **Sep-5** | NH | CF₃ | Cl | | | H | H | OH | H | OCH₂CH₂CH₂CH₂CH₃ | H | H |
| **37.** | **Sep-6** | NH | CF₃ | H | H | OCH₃ | H | H | OH | H | OCH₂Ph-4-NO₂ | H | H |
| **38.** | **Sep-7** | NH | CF₃ | H | H | OCH₃ | H | H | OH | H | OCH₂(CH₃)C=CH₂ | H | H |
| **39.** | **Sep-8** | NH | CF₃ | H | H | OCH₃ | H | H | OH | H | OCH₂Ph-2-F | H | H |
| **40.** | **Sep-9** | NH | CF₃ | H | H | H | H | H | OH | H | OCH₂(CH₃)C=CH₂ | H | H |
| **41.** | **Sep-10** | NH | CF₃ | H | H | H | H | H | OH | H | OCH₂Ph -4-CH=CH₂ | H | H |
| **42.** | **Sep-11** | NH | CF₃ | H | H | Cl | H | H | OH | H | OCH₂Ph-2-F, ₆-Cl | H | H |
| **43.** | **Sep-12** | NH | CF₃ | H | H | Cl | H | H | OH | H | OCH₂Ph-4-NO₂ | H | H |
| **44.** | **Sep-13** | NH | CF₃ | H | H | Cl | H | H | OH | H | OCH₂Ph-4-Br | H | H |
| **45.** | **Sep-14** | NH | CF₃ | H | H | Cl | H | H | OH | H | OCH₂Ph-4-F | H | H |
| **46.** | **Sep-15** | NH | CF₃ | OCH₃ | H | H | H | H | OH | H | OCH₂(CH₃)C=CH₂ | H | H |
| **47.** | **Sep-16** | NH | CF₃ | OCH₃ | H | H | H | H | OH | H | OCH₂Ph-4-CH₃ | H | H |
| **48.** | **Sep-17** | NH | CF₃ | OCH₃ | H | H | H | H | OH | H | OCH₂Ph | H | H |
| **49.** | **Sep-18** | NH | CF₃ | H | H | H | H | H | OH | H | OCH₂Ph-4-NO₂ | H | H |
| **50.** | **Sep-19** | NH | CF₃ | H | H | OCH₃ | H | H | OH | H | OCH₂Ph | H | H |
| **51.** | **Sep-20** | NH | CF₃ | H | H | Br | H | H | OH | H | OH | H | H |
| **52.** | **Sep-21** | NH | CH₃ | H | H | Cl | H | H | OH | H | OCH₃ | H | H |
| **53.** | **Sep-22** | NH | CH₃ | H | H | Cl | H | H | OH | H | OCH₂CH₃ | H | H |
| **54.** | **Sep-23** | NH | H | H | H | Cl | H | H | OH | H | OCH₃ | H | H |
| **55.** | **Sep-24** | NH | CF₃ | H | H | Cl | H | H | OH | H | OCH₂(CO)NHNH₂ | H | H |
| **56.** | **Sep-25** | NH | CF₃ | H | H | Cl | H | H | OH | H | OCH₂Ph-₂-Cl | H | H |
| **57.** | **Sep-26** | NH | CF₃ | OCH₃ | H | H | H | H | OH | H | OCH₂(CO)NHNH₂ | H | H |
| **58.** | **Sep-27** | NH | CF₃ | H | OCH₃ | OCH₃ | H | H | OH | H | OCH₂Ph-₄-CH₃ | H | H |
| **59.** | **Sep-28** | NH | CF₃ | H | OCH₃ | OCH₃ | H | H | OH | H | OCH₂Ph | H | H |
| **60.** | **Sep-29** | NH | CF₃ | H | H | Cl | H | H | OH | H | OCH₂(CH₃)C=CH₂ | H | H |
| **61.** | **Sep-30** | NH | CF₃ | OCH₃ | H | H | H | H | OH | H | OCH₂CH=CH(CH₃)₂ | H | H |

**Table 3: Preferred embodiments**

| Subst. | 1^{st} range | 2^{nd} range | 3^{rd} range | 4^{th} range | 5^{th} range |
|---|---|---|---|---|---|
| **X** | NH or O | NH | NH | NH | |
| **R₁** | H | H | H | H | |
| | C₁-C₄ alkyl , optionally substituted with one or more halogens, | C₁-C₂ alkyl, optionally substituted with one or more halogens, | C₁ alkyl optionally substituted with one or more halogens, | methyl or trifluoromethyl | methyl or trifluoromethyl |
| | wherein the halogen is selected from F, Cl, Br | wherein the halogen is selected from F, Cl, Br | wherein the halogen is selected from F, Cl, Br | wherein the halogen is F | wherein the halogen is F |
| **R₂** | H | H | H | H | H |
| | halogen selected from F, Cl, Br | halogen selected from F, Cl, Br | Cl | Cl | |
| | C₁-C₄ alkyl optionally substituted with one or more halogens, selected from F, Cl, Br | C₁-C₂-alkyl | C₁-alkyl | | |
| | C₁-C₄ alkoxy optionally substituted with one or more halogens, selected from F, Cl, Br | C₁-C₂ alkoxy | C₁-alkoxy | C₁-alkoxy | |
| **R₃** | H | H | H | H | |
| | F, Cl, Br, C₁-C₄ alkyl optionally substituted with one or more halogens, selected from F, Cl, Br | Cl, Br | Cl, Br | | |
| | C₁-C₄ alkoxy, optionally substituted with one or more halogens, selected from F, Cl, Br | C₁-C₂ alkoxy | C₁-alkoxy | C₁-alkoxy | |
| **R₄** | H | H | H | H | |
| | halogen selected from F, Cl, Br, C₁-C₄ alkyl optionally substituted with one or more halogens, selected from F, Cl, Br | halogen selected from F, Cl, Br, | halogen selected from F, Cl, Br, | Cl, Br | |
| | C₁-C₄ alkoxy optionally substituted with one or more halogens, selected from F, Cl, Br | C₁-C₄ alkoxy | C₁-C₂ alkoxy | C₁-alkoxy | |
| **R₅** | H | H | H | H | H |
| | halogen selected from F, Cl, Br, | halogen selected from F, Cl, Br, | Cl, Br | | |
| | C₁-C₄ alkyl optionally substituted with one or more halogens, selected from F, Cl, Br | C₁-C₄ alkyl | C₁-C₂-alkyl | C₁-alkyl | |
| | C₁-C₄ alkoxy optionally substituted with one or more halogens, selected from F, Cl, Br | C₁-C₄ alkoxy | C₁-C₂ alkoxy | C₁-alkoxy | |
| **R₆** | H | H | H | H | H |
| | halogen selected from F, Cl, Br | halogen selected from F, Cl, Br, | Cl, Br | | |
| | C₁-C₄ alkyl optionally substituted with one or more halogens, selected from F, Cl, Br | C₁-C₄ alkyl | C₁-C₂-alkyl | C₁-alkyl | |
| | C₁-C₄ alkoxy optionally substituted with one or more halogens, selected from F, Cl, Br | C₁-C₄ alkoxy | C₁-C₂ alkoxy | C₁-alkoxy | |
| **R₇** | H OR₁₂ | H OR₁₂ | H OR₁₂ | H OR₁₂ | H OR₁₂ |
| **R₈** | H | H | H | H | H |
| | halogen selected from F, Cl, Br | halogen selected from F, Cl, Br, | Cl, Br | | |
| | C₁-C₄ alkyl optionally substituted with one or more halogens, selected from F, Cl, Br | C₁-C₄ alkyl | C₁-C₂-alkyl | C₁-alkyl | |
| | C₁-C₄ alkoxy optionally substituted with one or more halogens, selected from F, Cl, Br | C₁-C₄ alkoxy | C₁-C₂ alkoxy | C₁-alkoxy | |
| **R₉** | H | H | H | H | H |
| | OR₁₃ | OR₁₃ | OR₁₃ | OR₁₃ | OR₁₃ |
| **R₁₀** | H | H | H | H | H |
| | halogen selected from F, Cl, Br, | halogen selected from F, Cl, Br, | Cl, Br | | |
| | C₁-C₄ alkyl optionally substituted with one or more halogens, selected from F, Cl, Br | C₁-C₄ alkyl | C₁-C₂-alkyl | C₁-alkyl | |
| | C₁-C₄ alkoxy optionally substituted with one or more halogens, selected from F, Cl, Br | C₁-C₄ alkoxy | C₁-C₂ alkoxy | C₁-alkoxy | |
| **R₁₁** | H | H | H | H | H |
| | halogen selected from F, Cl, Br, | halogen selected from F, Cl, Br, | Cl, Br | | |
| | C₁-C₄ alkyl optionally substituted with one or more halogens, selected from F, Cl, Br | C₁-C₄ alkyl | C₁-C₂-alkyl | C₁-alkyl | |
| | C₁-C₄ alkoxy optionally substituted with one or more halogens, selected from F, Cl, Br | C₁-C₄ alkoxy | C₁-C₂ alkoxy | C₁-alkoxy | |
| **R₁₂** | | | | | |
| **R₁₃** | H | H | H | H | H |
| | C₁-₈ alkyl | C₁-C₆ alkyl | C₁-C₂-Alkyl | C₁-C₂-Alkyl | C₁-C₂-Alkyl |
| | C2-C8 alkenyl | C2-C6-alkenyl | | | CH₂(CH₃)C=CH₂, CH₂CH=CH(CH₃)₂ |
| | CH₂Ph, the phenyl ring optionally being substituted with one or more of C₁-C₄-alkyl, C₂-C₄-alkenyl, NO₂, halogens selected from F, Cl, Br | phenyl ring being substituted with one or more of C₁-C₂-alkyl, C₁-C₄-alkenyl, NO₂, halogens selected from F, Cl, Br | phenyl ring being substituted with one or more of C₁-alkyl, C₂-alkenyl, NO₂, halogens selected from F, Cl, Br | phenyl ring being substituted with one or more of C₁-alkyl, C₂-alkenyl, NO₂, halogens selected from F, Cl, Br at the 2, 4 and/or 6 position | 2-F, 4-NO₂, 4-Cl, 4-Br 4-CH₃ 2-F-6-Cl, 4-CH=CH₂, 2-CI |

**Table 4: MIC and IC₅₀ results S. aureus Newman, S. suis, P. multocida, M. marinum, E. coli JW₅₅₀₃ and E. faecium Van A**

| | **MIC** | **MIC** | **MIC** | **MIC** | **MIC** | **MIC** | **IC50** | **IC50** | **IC50** |
|---|---|---|---|---|---|---|---|---|---|
| **Name** | ***S. aureus Newman*** | ***S. suis*** | ***P. multocida*** | ***M. marinum*** | ***E. coli JW5503*** | ***E. faecium Van A*** | **NRK-52E (50 mg/mL stock)** | **HEK293 (50 mg/mL stock)** | **HEK293 (5 mg/mL stock)** |
| **May-1** | 12.5 | 6.25 | 12.5 | 50 | 3.13 | 12.5 | 5.95 | | |
| **May-2** | 50 | 50 | >50 | >50 | 6.25 | 50 | 13.91 | | |
| **May-3** | 12.5 | 12.5 | >50 | 25 | 6.25 | 25 | ∼0.8 (no tox) | | 6.69 |
| **May-4** | 6.25 | 6.25 | 12.5 | 25 | 3.13 | 12.5 | ∼83.7 | | 8.96 |
| **May-5** | 25 | 50 | >50 | >50 | 12.5 | 50 | 6.11 | | |
| **May-6** | 1.56 | 3.13 | >50 | 50 | 3.13 | 3.13 | ∼563.4 (no tox) | | 4.53 |
| **May-7** | 6.25 | 6.25 | >50 | 50 | 3.13 | 12.5 | ∼31.15 | 230.8 | 5.68 |
| **May-8** | 12.5 | 12.5 | >50 | 50 | 3.13 | 25 | 2.64 | | |
| **May-9** | 12.5 | 12.5 | 50 | >50 | 3.13 | 25 | ∼3.15 | | |
| **May-10** | 6.25 | 12.5 | 25 | 50 | 1.56 | 12.5 | ∼3.61 | | |
| **May-11** | 1.56 | 3.13 | >50 | 12.5 | >50 | 1.56 | ∼3.91 | 6.2 | |
| **May-12** | 12.5 | 6.25 | 50 | >50 | 6.25 | 12.5 | 27.17 | | 7.04 |
| **May-13** | 25 | 25 | 50 | >50 | 3.13 | 50 | 8.06 | | |
| **May-14** | 3.13 | 12.5 | 25 | >50 | 3.13 | 12.5 | 3.04 | 10.6 | |
| **May-15** | 1.56 | 3.13 | >50 | 12.5 | >50 | 3.13 | 5.13 | 6.9 | |
| **May-16** | 3.13 | 3.13 | 25 | 50 | 1.56 | 6.25 | 5.782E-07 | | 3.59 |
| **May-17** | 25 | 25 | >50 | >50 | 6.25 | 25 | 22.03 | | 14.98 |
| **May-18** | 0.78 | 3.13 | >50 | 6.25 | >50 | 3.13 | 4.568 | 4.7 | |
| **May-19** | 25 | 25 | 50 | 50 | 6.25 | >50 | 24.09 | | 5.74 |
| **May-20** | 6.25 | 6.25 | 50 | 25 | 3.13 | 12.5 | ∼17.6 | | 2.10 |
| **May-21** | 12.5 | 25 | 50 | >50 | 3.13 | 25 | ∼15.64 | 46.9 | 6.47 |
| **May-22** | 3.13 | 6.25 | 50 | >50 | 1.56 | >50 | 19.17 | 19.9 | 5.80 |
| **May-23** | 3.13 | 6.25 | 50 | >50 | 3.13 | 3.13 | ∼2.814 | 9.0 | 8.38 |
| **May-24** | 1.56 | 3.13 | >50 | 25 | 25 | 1.56 | ∼3.86 | 4.8 | |
| **May-25** | 12.5 | | 50 | | 6.25 | 25 | 106.2 | | 27.60 |
| **May-26** | 1.56 | 3.13 | >50 | 12.5 | 3.13 | 1.56 | 9.64 | 4.8 | |
| **May-27** | 3.13 | 3.13 | 25 | 50 | 3.13 | 3.13 | 180.8 | 118.3 | 10.82 |
| **Sep-1** | 25 | 50 | >50 | >50 | 6.25 | 50 | 23.19 | | |
| **Sep-2** | 6.25 | 25 | >50 | >50 | 25 | 25 | ∼17.39 | | 3.97 |
| **Sep-3** | 12.5 | 25 | >50 | >50 | 6.25 | 25 | ∼15.5 | | |
| **Sep-4** | 3.13 | 25 | >50 | 50 | >50 | 25 | 13.87 | | |
| **Sep-5** | 3.13 | 12.5 | >50 | 50 | >50 | 12.5 | ∼7.46 | | |
| **Sep-6** | 3.13 | 12.5 | >50 | 50 | 12.5 | 6.25 | 8.42 | | |
| **Sep-7** | 6.25 | 12.5 | >50 | >50 | 6.25 | 12.5 | ∼7.49 | | |
| **Sep-8** | 3.13 | 12.5 | >50 | >50 | 50 | 6.25 | ∼14.53 | | |
| **Sep-9** | 6.25 | 25 | 50 | >50 | 6.25 | 0.78 | ∼10.87 | | 16.12 |
| **Sep-11** | 3.13 | 12.5 | >50 | >50 | >50 | 6.25 | 11.84 | | |
| **Sep-12** | 3.13 | 12.5 | >50 | 50 | >50 | 12.5 | 14.72 | | |
| **Sep-13** | 1.56 | 6.25 | >50 | 25 | >50 | 12.5 | 10.89 | | 16.51 |
| **Sep-14** | 12.5 | 25 | >50 | >50 | 25 | 12.5 | ∼16.64 | | |
| **Sep-15** | 3.13 | 12.5 | >50 | 50 | 6.25 | 6.25 | ∼7.62 | | |
| **Sep-16** | 6.25 | 25 | >50 | >50 | 50 | 12.5 | ∼8.72 | | |
| **Sep-17** | 6.25 | 25 | >50 | >50 | 12.5 | 12.5 | ∼8.97 | | |
| **Sep-18** | 6.25 | 12.5 | >50 | >50 | 12.5 | 12.5 | ∼14.73 | | |
| **Sep-20** | 6.25 | 25 | >50 | >50 | 6.25 | 25 | 7.58 | | |
| **Sep-21** | 50 | >50 | >50 | 50 | | 50 | 18.24 | | |
| **Sep-22** | 50 | 50 | >50 | >50 | | >50 | ∼1483(?) | 23.6 | |
| **Sep-23** | 25 | >50 | >50 | >50 | | >50 | 259 | 39.7 | 9.14 |
| **Sep-24** | 50 | >50 | >50 | >50 | | 50 | 103.5 | 119.0 | |
| **Sep-25** | 3.13 | 12.5 | >50 | 50 | >50 | 12.5 | 17.27 | 18.05 | 19.29 |
| **Sep-26** | >50 | >50 | >50 | >50 | | 25 | 194.5 | | |
| **Sep-27** | 3.13 | 50 | >50 | 50 | >50 | >50 | ∼17.59 | | |
| **Sep-28** | 12.5 | >50 | >50 | >50 | 25 | >50 | ∼17.27 | | |
| **Sep-29** | 3.13 | 12.5 | >50 | >50 | 12.5 | 6.25 | 19.91 | | 16.39 |
| **Sep-30** | 6.25 | 25 | >50 | >50 | 6.25 | 12.5 | ∼17.23 | | |
| **AND-1** | 3.13 | 12.5 | 25 | >50 | 1.56 | 12.5 | ∼3.86 | 14.7 | 6.94 |
| **AND-2** | 3.13 | 12.5 | 25 | >50 | 1.56 | 6.25 | ∼3.88 | 36.7 | 6.97 |
| **AND-3** | 25 | 50 | 50 | >50 | 6.25 | 0.78 | ∼354.2 | 62.9 | 12.19 |
| **AND-4** | 3.13 | 12.5 | 25 | >50 | 3.13 | 12.5 | ∼3.95 | 6.2 | 5.33 |

**Table 5: Selectivity Index S. Aureus Newman. S. Suis and P multocida**

| **Name** | **NRK-52E *S. aureus Newman*** | **HEK 293 *S*. *aureus Newman*** | **HEK 293 (5 mg/mL stock) *S*. *aureus Newman*** | **NRK-52E *S. suis*** | **HEK 293 *S*. *suis*** | **HEK 293 *S*. *suis* (5 mg/mL stock)** | **NRK-52E *P. multocida*** | **HEK 293 *P multocida*** | **HEK 293 *P. multocida* (5 mg/mL stock)** |
|---|---|---|---|---|---|---|---|---|---|
| **May-1** | 0.48 | | | 0.95 | | | 0.48 | | |
| **May-2** | 0.28 | | | 0.28 | | | <0.278 | | |
| **May-3** | 40.00 | | 0.54 | 40.00 | | 0.54 | >10 | | <0.13 |
| **May-4** | 13.39 | | 1.43 | 13.39 | | 1.43 | 6.70 | | 0.72 |
| **May-5** | 0.24 | | . | 0.12 | | . | <0.12 | | . |
| **May-6** | 361.15 | | 2.90 | 180.10 | | 1.45 | <11.27 | | <0.09 |
| **May-7** | 4.98 | 36.93 | 0.91 | 4.98 | 36.93 | 0.91 | <0.62 | <4.62 | <0.11 |
| **May-8** | 0.21 | | . | 0.21 | | . | <0.05 | | . |
| **May-9** | 0.25 | | . | 0.25 | | . | 0.06 | | . |
| **May-10** | 0.58 | | . | 0.29 | | . | 0.14 | | . |
| **May-11** | 2.51 | 3.97 | . | 1.25 | 1.98 | . | <0.078 | <0.124 | . |
| **May-12** | 2.17 | | 0.56 | 4.35 | | 1.13 | 0.54 | | 0.14 |
| **May-13** | 0.32 | | . | 0.32 | | . | 0.16 | | . |
| **May-14** | 0.97 | 3.39 | . | 0.24 | 0.85 | . | 0.12 | 0.42 | . |
| **May-15** | 3.29 | 4.42 | . | 1.64 | 2.20 | . | <0.10 | <0.14 | . |
| **May-16** | 0.00 | | 1.15 | 0.00 | | 1.15 | 0.00 | | 0.14 |
| **May-17** | 0.88 | | 0.60 | 0.88 | | 0.60 | <0.4406 | | <0.30 |
| **May-18** | 5.86 | 6.03 | . | 1.46 | 1.50 | . | <0.091 | <0.094 | . |
| **May-19** | 0.96 | | 0.23 | 0.96 | | 0.23 | 0.48 | | 0.11 |
| **May-20** | 2.82 | | 0.34 | 2.82 | | 0.34 | 0.35 | | 0.04 |
| **May-21** | 1.25 | 3.75 | 0.52 | 0.63 | 1.88 | 0.26 | 0.31 | 0.94 | 0.13 |
| **May-22** | 6.12 | 6.36 | 1.85 | 3.07 | 3.18 | 0.93 | 0.38 | 0.40 | 0.12 |
| **May-23** | 0.90 | 2.88 | 2.68 | 0.45 | 1.44 | 1.34 | 0.06 | 0.18 | 0.17 |
| **May-24** | 2.47 | 3.08 | . | 1.23 | 1.53 | . | <0.07 | <0.096 | . |
| **May-25** | 8.50 | | 2.21 | . | | . | 2.12 | | 0.55 |
| **May-26** | 6.18 | 3.08 | . | 3.08 | 1.54 | . | <0.1928 | <0.096 | . |
| **May-27** | 57.76 | 37.80 | 3.46 | 57.76 | 37.80 | 3.46 | 7.23 | 4.73 | 0.43 |
| **Sep-1** | 0.93 | | . | 0.46 | | . | <0.46 | | . |
| **Sep-2** | 2.78 | | 0.64 | 0.70 | | 0.16 | <0.35 | | <0.08 |
| **Sep-3** | 1.24 | | . | 0.62 | | . | <0.31 | | . |
| **Sep-4** | 4.43 | | . | 0.55 | | . | <0.28 | | . |
| **Sep-5** | 2.38 | | . | 0.60 | | . | <0.15 | | . |
| **Sep-6** | 2.69 | | . | 0.67 | | . | <0.17 | | . |
| **Sep-7** | 1.20 | | . | 0.60 | | . | <0.15 | | . |
| **Sep-8** | 4.64 | | . | 1.16 | | . | <0.29 | | . |
| **Sep-9** | 1.74 | | 2.58 | 0.43 | | 0.64 | 0.22 | | 0.32 |
| **Sep-11** | 3.78 | | . | 0.95 | | . | <0.24 | | . |
| **Sep-12** | 4.70 | | . | 1.18 | | . | <0.29 | | . |
| **Sep-13** | 6.98 | | 10.58 | 1.74 | | 2.64 | <0.22 | | <0.33 |
| **Sep-14** | 1.33 | | . | 0.67 | | . | <0.33 | | . |
| **Sep-15** | 2.43 | | . | 0.61 | | . | <0.15 | | . |
| **Sep-16** | 1.40 | | . | 0.35 | | . | <0.17 | | . |
| **Sep-17** | 1.44 | | . | 0.36 | | . | <0.18 | | . |
| **Sep-18** | 2.36 | | . | 1.18 | | . | <0.30 | | . |
| **Sep-20** | 1.21 | | . | 0.30 | | . | <0.15 | | . |
| **Sep-21** | 0.36 | | . | <0.36 | | . | <0.36 | | . |
| **Sep-22** | 10.00 | 0.47 | . | 10.00 | 0.47 | . | <10 | <0.47 | . |
| **Sep-23** | 10.36 | 1.59 | 0.37 | <5.18 | <0.79 | <0.18 | <5.18 | <0.79 | <0.18 |
| **Sep-24** | 2.07 | 2.38 | . | <2.07 | <2.38 | . | <2.07 | <2.38 | . |
| **Sep-25** | 5.52 | 5.77 | 6.16 | 1.38 | 1.44 | 1.54 | <0.35 | <0.36 | <0.39 |
| **Sep-26** | <3.88 | | . | <3.88 | | . | <3.88 | | . |
| **Sep-27** | 5.62 | | . | 0.35 | | . | <0.35 | | . |
| **Sep-28** | 1.38 | | . | <0.36 | | . | <0.36 | | . |
| **Sep-29** | 6.36 | | 5.24 | 1.59 | | 1.31 | <0.40 | | 0.33 |
| **Sep-30** | 2.76 | | . | 0.69 | | . | <0.34 | | . |
| **AND-1** | 1.23 | 4.70 | 2.22 | 0.31 | 1.18 | 0.56 | 0.15 | 0.59 | 0.28 |
| **AND-2** | 1.24 | 11.73 | 2.23 | 0.31 | 2.94 | 0.56 | 0.16 | 1.47 | 0.28 |
| **AND-3** | 14.17 | 2.52 | 0.49 | 7.08 | 1.26 | 0.24 | 7.08 | 1.26 | 0.24 |
| **AND-4** | 1.26 | 1.98 | 1.70 | 0.32 | 0.50 | 0.43 | 0.16 | 0.25 | 0.21 |

**Table 6: Selectivity Index M. marinum, E. coli JW₅₅₀₃, and E. faecium Van A**

| **Name** | **NRK-52E *M. marinum*** | **HEK 293 *M. Marinum*** | **HEK 293 *M. Marinum* (5 mg/mL stock)** | **NRK-52E *E. coli JW5503*** | **HEK 293 *E. coli JW5503*** | **HEK 293 *E. coli JW5503* (5 mg/mL stock)** | **NRK-52E *E. faecium Van A*** | **HEK 293 *E. faecium VanA*** | **HEK 293 *E. faecium VanA* (5 mg/mL stock)** |
|---|---|---|---|---|---|---|---|---|---|
| **May-1** | 0.12 | | | 1.90 | | | 0.48 | | |
| **May-2** | <0.278 | | | 2.23 | | | 0.28 | | |
| **May-3** | 20.00 | | 0.27 | 80.00 | | 1.07 | 40.00 | | 0.27 |
| **May-4** | 3.35 | | 0.36 | 26.74 | | 2.86 | 6.70 | | 0.72 |
| **May-5** | <0.12 | | . | 0.49 | | . | 0.12 | | . |
| **May-6** | 11.27 | | 0.09 | 180.10 | | 1.45 | 180.10 | | 1.45 |
| **May-7** | 0.62 | 4.62 | 0.11 | 9.95 | 73.74 | 1.81 | 2.49 | 18.46 | 0.45 |
| **May-8** | 0.05 | | . | 0.84 | | . | 0.11 | | . |
| **May-9** | <0.063 | | . | 1.01 | | . | 0.13 | | . |
| **May-10** | 0.07 | | . | 2.31 | | . | 0.29 | | . |
| **May-11** | 0.31 | 0.50 | . | <0.078 | <0.124 | . | 2.51 | 3.97 | . |
| **May-12** | <0.5434 | | <0.27 | 4.35 | | 1.13 | 2.17 | | 0.56 |
| **May-13** | <0.1612 | | . | 2.58 | | . | 0.16 | | . |
| **May-14** | <0.061 | <0.21 | . | 0.97 | 3.39 | . | 0.24 | 0.83 | . |
| **May-15** | 0.41 | 0.55 | . | <0.10 | <1.14 | . | 1.64 | 2.2 | . |
| **May-16** | 0.00 | | 0.07 | 0.00 | | 2.30 | 0.00 | | 0.57 |
| **May-17** | <0.4406 | | <0.30 | 3.52 | | 2.40 | 0.88 | | 0.60 |
| **May-18** | 0.73 | 0.75 | . | <0.091 | <0.094 | . | 1.46 | 1.5 | . |
| **May-19** | 0.48 | | 0.11 | 3.85 | | 0.92 | <0.48 | | <0.11 |
| **May-20** | 0.70 | | 0.08 | 5.62 | | 0.67 | 1.41 | | 0.17 |
| **May-21** | <0.31 | <0.94 | <0.13 | 5.00 | 15.00 | 2.07 | 0.63 | 1.88 | 0.26 |
| **May-22** | <0.38 | <0.40 | <0.12 | 12.29 | 12.76 | 3.72 | <0.38 | <0.40 | <0.12 |
| **May-23** | <0.06 | <0.18 | <0.17 | 0.90 | 2.88 | 2.68 | 0.90 | 2.88 | 2.68 |
| **May-24** | 0.15 | 0.19 | . | 0.15 | 0.19 | . | 2.47 | 3.08 | . |
| **May-25** | . | | . | 16.99 | | 4.42 | 4.25 | | 1.10 |
| **May-26** | 0.77 | | . | 3.08 | 1.54 | . | 6.18 | 3.08 | . |
| **May-27** | 3.62 | 2.37 | 0.22 | 57.76 | 37.80 | 3.46 | 57.76 | 37.8 | 3.46 |
| **Sep-1** | <0.46 | | . | 3.71 | | . | 0.46 | | . |
| **Sep-2** | <0.35 | | <0.08 | 0.70 | | 0.16 | 0.70 | | 0.16 |
| **Sep-3** | <0.31 | | . | 2.48 | | . | 0.62 | | . |
| **Sep-4** | 0.28 | | . | <0.28 | | . | 0.55 | | . |
| **Sep-5** | 0.15 | | . | <0.15 | | . | 0.60 | | . |
| **Sep-6** | 0.17 | | . | 0.67 | | . | 1.35 | | . |
| **Sep-7** | <0.15 | | . | 1.20 | | . | 0.60 | | . |
| **Sep-8** | <0.29 | | . | 0.29 | | . | 2.32 | | . |
| **Sep-9** | <0.22 | | <0.32 | 1.74 | | 2.58 | 13.94 | | 20.67 |
| **Sep-11** | <0.24 | | . | <0.24 | | . | 1.89 | | . |
| **Sep-12** | 0.29 | | . | <0.29 | | . | 1.18 | | . |
| **Sep-13** | 0.44 | | 0.66 | <0.22 | | <0.33 | 0.87 | | 1.32 |
| **Sep-14** | <0.33 | | . | 0.67 | | . | 1.33 | | . |
| **Sep-15** | 0.15 | | . | 1.22 | | . | 1.22 | | . |
| **Sep-16** | <0.17 | | . | 0.17 | | . | 0.70 | | . |
| **Sep-17** | <0.18 | | . | 0.72 | | . | 0.72 | | . |
| **Sep-18** | <0.30 | | . | 1.18 | | . | 1.18 | | . |
| **Sep-20** | <0.15 | | . | 1.21 | | . | 0.30 | | . |
| **Sep-21** | 0.36 | | . | . | | . | 0.36 | | . |
| **Sep-22** | <10 | <0.47 | . | . | | . | <10 | <0.47 | . |
| **Sep-23** | <5.18 | <0.79 | <0.18 | . | | . | <5.18 | <0.79 | <0.18 |
| **Sep-24** | <2.07 | <2.38 | . | . | | . | 2.07 | 2.38 | . |
| **Sep-25** | 0.35 | 0.36 | 0.39 | <0.35 | <0.36 | <0.39 | 1.38 | 1.44 | 1.54 |
| **Sep-26** | <3.88 | | . | . | | . | 7.78 | | . |
| **Sep-27** | 0.35 | | . | <0.35 | | . | <0.35 | | . |
| **Sep-28** | <0.36 | | . | 0.69 | | . | <0.36 | | . |
| **Sep-29** | <0.40 | | <0.33 | 1.59 | | 1.31 | 3.19 | | 2.62 |
| **Sep-30** | <0.34 | | . | 2.76 | | . | 1.38 | | . |
| **AND-1** | <0.077 | <0.29 | <0.14 | 2.47 | 9.42 | 4.45 | 0.31 | 1.18 | 0.56 |
| **AND-2** | <0.078 | <0.73 | <0.14 | 2.49 | 23.53 | 4.47 | 0.62 | 5.87 | 1.12 |
| **AND-3** | <7.08 | <1.26 | <0.24 | 56.67 | 10.06 | 1.95 | 454.10 | 80.64 | 15.63 |
| **AND-4** | <0.079 | <0.125 | <0.11 | 1.26 | 1.98 | 1.70 | 0.32 | 0.5 | 0.43 |

## Claims

1. Use of a compound of formula (I) or a pharmaceutically acceptable salt or solvate thereof as an antibacterial 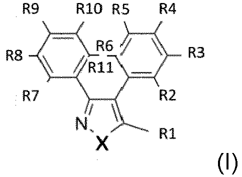 wherein
**X** is NH or O;
**R1** is H;
C₁-C₄ alkyl, optionally substituted with one or more halogens wherein the halogen is selected from F, Cl, Br;
**R2** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R3** is H;
F, Cl, Br;
C₁-C₄ alkyl optionally substituted with one or more halogens, selected from F, Cl, Br;
C₁-C₄ alkoxy, optionally substituted with one or more halogens, selected from F, Cl, Br;
**R4** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R5** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R6** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R7** is H;
OR12;
**R8** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R9** is H;
OR13;
**R10** is H; halogen selected from F, Cl, Br; C₁-C₄ alkyl optionally substituted with one or more halogens, selected from F, Cl, Br; C₁-C₄ alkoxy optionally substituted with one or more halogens, selected from F, Cl, Br;
**R11** is H;
halogen selected from F, Cl, Br;
C₁-C₄ alkyl optionally substituted with one or more halogens, selected from F, Cl, Br;
C₁-C₄ alkoxy optionally substituted with one or more halogens, selected from F, Cl, Br;
**R12** is H;
C₁-C₄ alkyl;
**R13** is H;
C₁-8 alkyl ;
C2-C8 alkenyl;
CH2Ph, the phenyl ring optionally being substituted with one or more of C₁-C₄-alkyl, C₂-C₄-alkenyl, NO2, halogens selected from F, Cl, Br;

2. Use according to claim 1, wherein
**X** is NH
**R1** is H
C₁-C₂ alkyl, optionally substituted with one or more halogens, wherein the halogen is selected from F, Cl, Br
**R2** is H
halogen selected from F, Cl, Br
C₁-C₂-alkyl
C₁-C₂ alkoxy
**R3** is H
Cl, Br
C₁-C₂ alkoxy
**R4** is H
halogen selected from F, Cl, Br,
C₁-C₄ alkoxy
**R5** is H
halogen selected from F, Cl, Br,
C₁-C₄ alkyl
C₁-C₄ alkoxy
**R6** is H
halogen selected from F, Cl, Br,
C₁-C₄ alkyl
C₁-C₄ alkoxy
**R7** is H
OR12
**R8** is H
halogen selected from F, Cl, Br,
C₁-C₄ alkyl
C₁-C₄ alkoxy
**R9** is H
OR13
**R10** is H
halogen selected from F, Cl, Br, C₁-C₄ alkyl C₁-C₄ alkoxy
**R11** is H
halogen selected from F, Cl, Br,
C₁-C₄ alkyl
C₁-C₄ alkoxy
**R12** is H
C₁-C₂-alkyl
**R13** is H
C₁-C6 alkyl
C₂-C6-alkenyl phenyl ring being substituted with one or more of C₁-C₂-alkyl, C₁-C₄-alkenyl, NO2, halogens selected from F, Cl, Br

3. Use according to claim 1 or 2, wherein
**X** is NH
**R1** is H
C₁ alkyl optionally substituted with one or more halogens, wherein the halogen is selected from F, Cl, Br
**R2** is H
Cl
C₁-alkyl
C₁-alkoxy
**R3** is H
Cl, Br
C₁-alkoxy
**R4** is H
halogen selected from F, Cl, Br, C₁-C₂ alkoxy
**R5** is H
Cl, Br
C₁-C₂-alkyl
C₁-C₂ alkoxy
**R6** is H
Cl, Br
C₁-C₂-alkyl
C₁-C₂ alkoxy
**R7** is H
OR12
**R8** is H
Cl, Br
C₁-C₂-alkyl
C₁-C₂ alkoxy
**R9** is H
OR13
**R10** is H
Cl, Br
C₁-C₂-alkyl
C₁-C₂ alkoxy
**R11** is H
CI, Br
C₁-C₂-alkyl
C₁-C₂ alkoxy
**R12** is H
**R13** is H
C₁-C₂-Alkyl
phenyl ring being substituted with one or more of C₁-alkyl, C₂-alkenyl, NO2, halogens selected from F, Cl, Br

4. Use of a compound or a pharmaceutically acceptable salt or solvate thereof as an antibacterial wherein the compound selected from the list consisting of the following compounds in SMILES representations:
- OC1ccc(-C2[nH]nc(C2-C2ccc(Br)cC2)C(F)(F)F)c(O)C1;
- OC1ccc(-C2onc(C2-C2ccc(Cl)cC2)C(F)(F)F)c(O)C1;
- CC1noc(C1-C1ccc(Br)cC1)-C1ccc(O)cC1O;
- CC1[nH]nc(C1-C1ccc(Br)cC1)-C1ccc(O)cC1O;
- COC1ccc(cC1OC)-C1c([nH]nC1C(F)(F)F)-C1ccc(O)cC1O;
- CCOC1ccc(-C2[nH]nc(C2-C2ccc(OC)c(OC)C2)C(F)(F)F)c(O)C1;
- COC1ccc(-C2[nH]nc(C)C2-C2ccc(Br)cC2)c(O)C1;
- COC1ccc(-C2[nH]nc(C2-C2ccc(OC)c(OC)C2)C(F)(F)F)c(O)C1;
- CCOC1ccc(-C2[nH]nc(C2-C2ccc(Br)cC2)C(F)(F)F)c(O)C1;
- CCOC1ccc(-C2n[nH]c(C2-C2ccc(OC)cC2)C(F)(F)F)c(O)C1;
- COC1ccc(cC1)-C1c(n[nH]C1C(F)(F)F)-C1ccc(OC)cC1O;
- COC1ccc(cC1)-C1c(n[nH]C1C(F)(F)F)-C1ccc(O)cC1O;
- OC1ccc(-C2[nH]nc(C2-C2ccccC2)C(F)(F)F)c(O)C1;
- OC1cc(OCC2ccc(cC2)[N+]([O-])=O)ccC1-C1[nH]nc(C1-C1ccc(Br)cC1)C(F)(F)F;
- NNC(=O)COC1ccc(-C2[nH]nc(C2-C2ccc(Br)cC2)C(F)(F)F)c(O)C1;
- COC1ccccC1-C1c([nH]nC1C(F)(F)F)-C1ccc(O)cC1O;
- OC1ccc(-C2n[nH]c(C2-C2ccc(Cl)cC2)C(F)(F)F)c(O)C1;
- OC1cc(OCC2ccccC2)ccC1-C1n[nH]c(C1-C1ccc(Br)cC1)C(F)(F)F;
- COC1ccc(-C2n[nH]c(C2-C2ccc(Cl)cC2)C(F)(F)F)c(O)C1;
- COC1ccc(cC1)-C1c(n[nH]C1C(F)(F)F)-C1ccc(OCC(=O)NN)cC1O;
- OC1cc(OCC2ccc(Br)cC2)ccC1-C1[nH]nc(C1-C1ccc(CI)cC1)C(F)(F)F;
- COC1ccc(-C2n[nH]c(C2-C2ccccC2OC)C(F)(F)F)c(O)C1;
- CCOC1ccc(-C2n[nH]c(C2-C2ccccC2OC)C(F)(F)F)c(O)C1;
- COC1ccc(-C2[nH]nc(C2-C2ccccC2)C(F)(F)F)c(O)C1;
- CCOC1ccc(-C2n[nH]c(C2-C2ccccC2)C(F)(F)F)c(O)C1;
- COC1ccc(-C2[nH]nc(C2-C2ccc(Br)cC2)C(F)(F)F)c(O)C1;
- OC1cc(OCC2ccccC2)ccC1-C1[nH]nc(C1-C1ccccC1)C(F)(F)F;
- NNC(=O)COC1ccc(-C2[nH]nc(C2-C2ccccC2)C(F)(F)F)c(O)C1;
- COC1ccc(cC1)-C1c(n[nH]C1-C1ccc(OCC2ccccC2)cC1O)C(F)(F)F;
- CCOC1ccc(-C2[nH]nc(C2-C2ccc(Cl)cC2)C(F)(F)F)c(O)C1;
- OC1ccc(-C2n[nH]c(C2-C2ccc(F)cC2)C(F)(F)F)c(O)C1;
- COC1ccc(cC1)-C1c(n[nH]C1C(F)(F)F)-C1ccc(OC)c(C)C1O;
- CCOC1cc(O)c(cC1CC)-C1n[nH]c(C1-C1ccccC1)C(F)(F)F;
- CCOC1ccc(-C2n[nH]c(C2-C2ccccC2)C(F)(F)F)c(O)C1C;
- CC1ccc(COC2ccc(-c3n[nH]c(c3-c3cccC4ccccc34)C(F)(F)F)c(O)C2)cC1;
- CCCCCOC1ccc(-C2n[nH]c(C2-C2ccccC2Cl)C(F)(F)F)c(O)C1;
- COC1ccc(cC1)-C1c(n[nH]C1-C1ccc(OCC2ccc(cC2)[N+]([O-])=O)cC1O)C(F)(F)F;
- COC1ccc(cC1)-C1c(n[nH]C1-C1ccc(OCC(C)=C)cC1O)C(F)(F)F;
- COC1ccc(cC1)-C1c(n[nH]C1-C1ccc(OCC2ccccC2F)cC1O)C(F)(F)F;
- CC(=C)COC1ccc(-C2[nH]nc(C2-C2ccccC2)C(F)(F)F)c(O)C1;
- OC1cc(OCC2ccc(C=C)cC2)ccC1-C1[nH]nc(C1-C1ccccC1)C(F)(F)F;
- OC1cc(OCC2c(F)cccC2CI)ccC1-C1[nH]nc(C1-C1ccc(Cl)cC1)C(F)(F)F;
- OC1cc(OCC2ccc(cC2)[N+]([O-])=O)ccC1-C1[nH]nc(C1-C1ccc(Cl)cC1)C(F)(F)F;
- OC1cc(OCC2ccc(Br)cC2)ccC1-C1[nH]nc(C1-C1ccc(Cl)cC1)C(F)(F)F;
- COC1ccc(cC1)-C1c(n[nH]C1-C1ccc(OCC2ccc(F)cC2)cC1O)C(F)(F)F;
- COC1ccccC1-C1c(n[nH]C1-C1ccc(OCC(C)=C)cC1O)C(F)(F)F;
- COC1ccccC1-C1c(n[nH]C1-C1ccc(OCC2ccc(C)cC2)cC1O)C(F)(F)F;
- COC1ccccC1-C1c(n[nH]C1-C1ccc(OCC2ccccC2)cC1O)C(F)(F)F;
- OC1cc(OCC2ccc(cC2)[N+]([O-])=O)ccC1-C1[nH]nc(C1-C1ccccC1)C(F)(F)F;
- COC1ccc(cC1)-C1c(n[nH]C1-C1ccc(OCC2ccccC2)cC1O)C(F)(F)F;
- OC1ccc(-C2n[nH]c(C2-C2ccc(Br)cC2)C(F)(F)F)c(O)C1;
- COC1ccc(-C2n[nH]c(C)C2-C2ccc(Cl)cC2)c(O)C1;
- CCOC1ccc(-C2n[nH]c(C)C2-C2ccc(Cl)cC2)c(O)C1;
- COC1ccc(-C2n[nH]cC2-C2ccc(Cl)cC2)c(O)C1;
- NNC(=O)COC1ccc(-C2n[nH]c(C2-C2ccc(Cl)cC2)C(F)(F)F)c(O)C1;
- OC1cc(OCC2ccccC2Cl)ccC1-C1n[nH]c(C1-C1ccc(Cl)cC1)C(F)(F)F;
- COC1ccccC1-C1c(n[nH]C1C(F)(F)F)-C1ccc(OCC(=O)NN)cC1O;
- COC1ccc(cC1OC)-C1c(n[nH]C1-C1ccc(OCC2ccc(C)cC2)cC1O)C(F)(F)F;
- COC1ccc(cC1OC)-C1c(n[nH]C1-C1ccc(OCC2ccccC2)cC1O)C(F)(F)F;
- CC(=C)COC1ccc(-C2[nH]nc(C2-C2ccc(Cl)cC2)C(F)(F)F)c(O)C1;
- COC1ccccC1-C1c(n[nH]C1-C1ccc(OCC=C(C)C)cC1O)C(F)(F)F.

5. Use according to claims 1-4, wherein the compound of formula I is selected from the group consisting of: 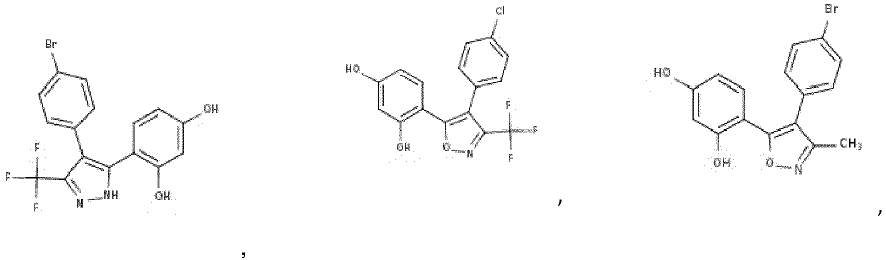 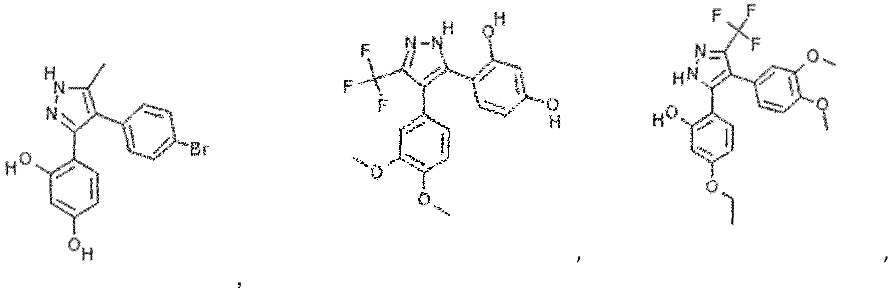 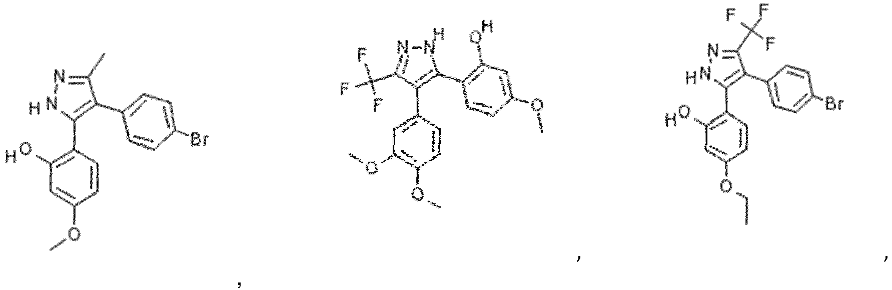 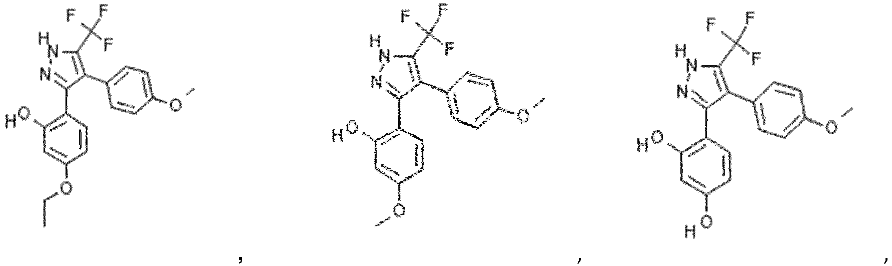 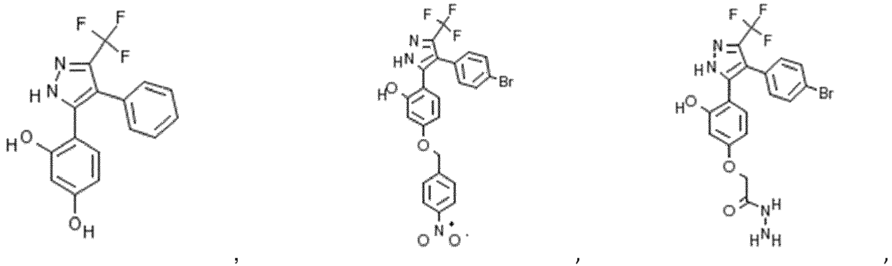 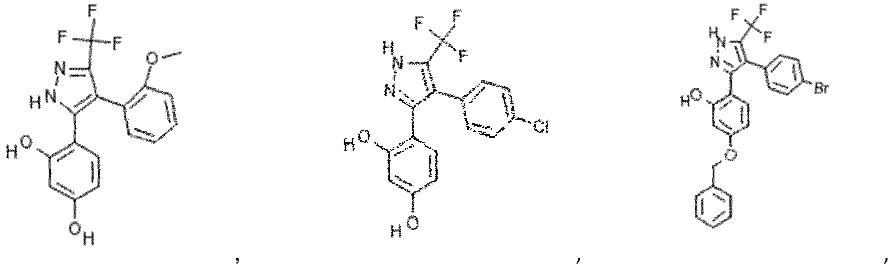 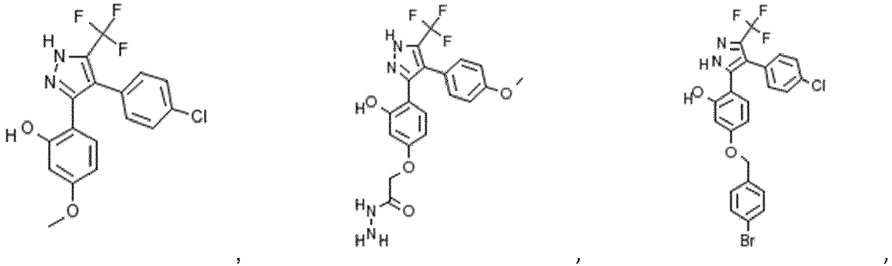 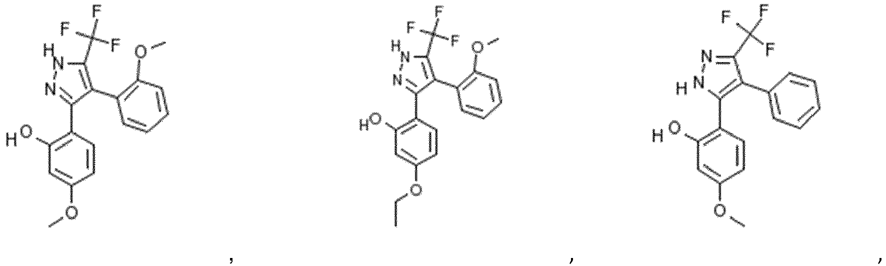 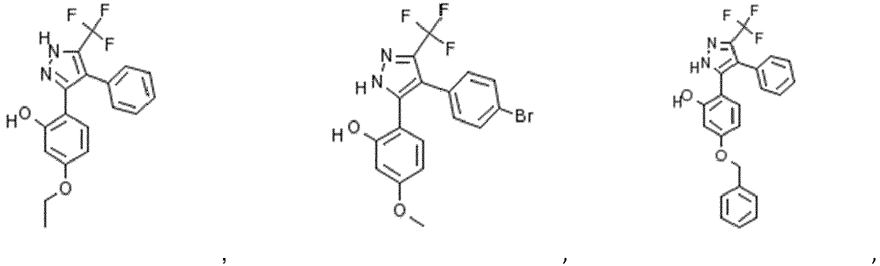 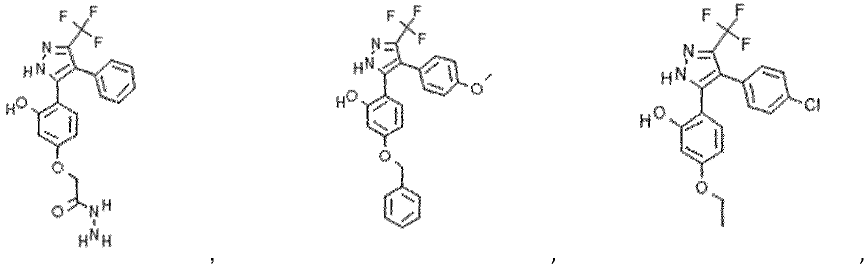 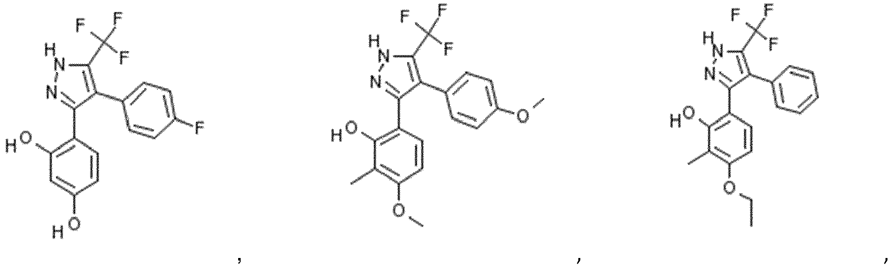 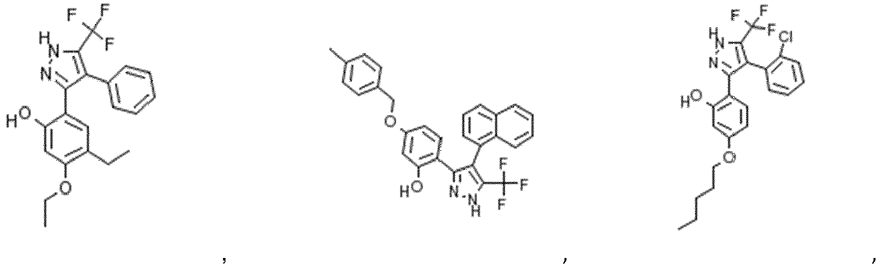 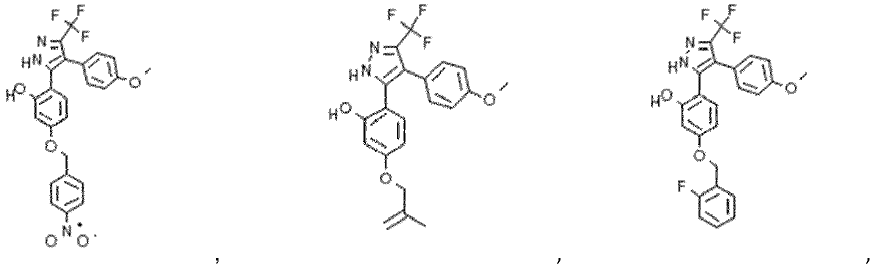 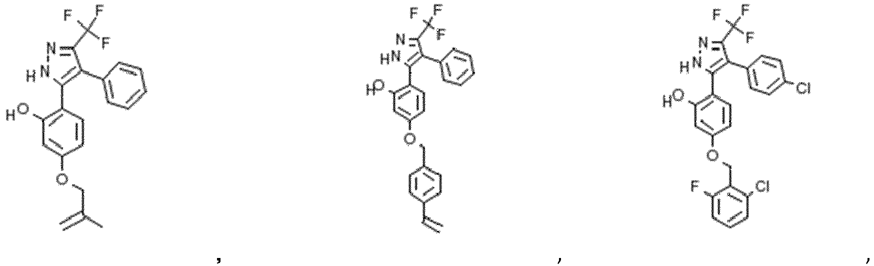 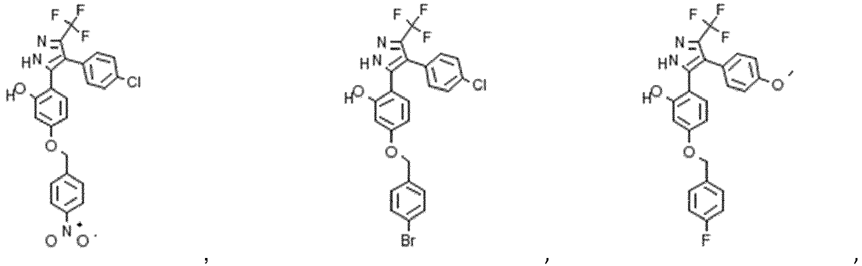 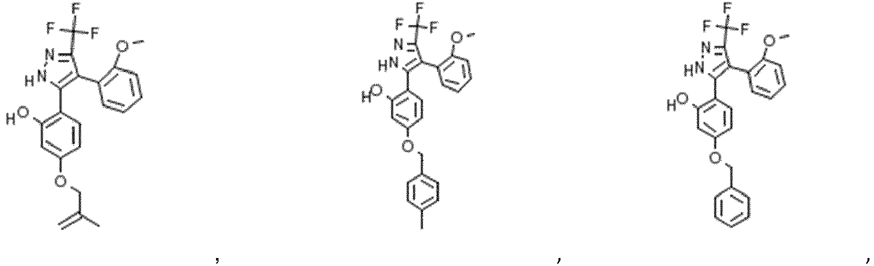 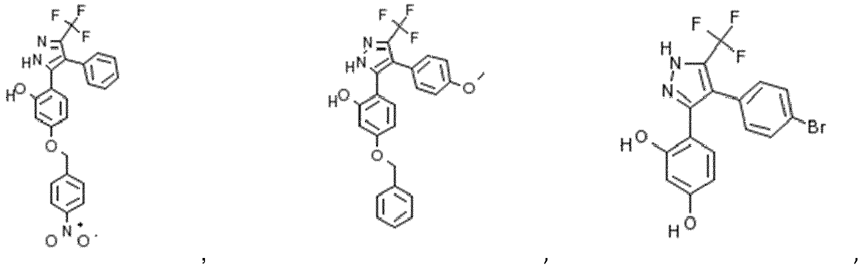 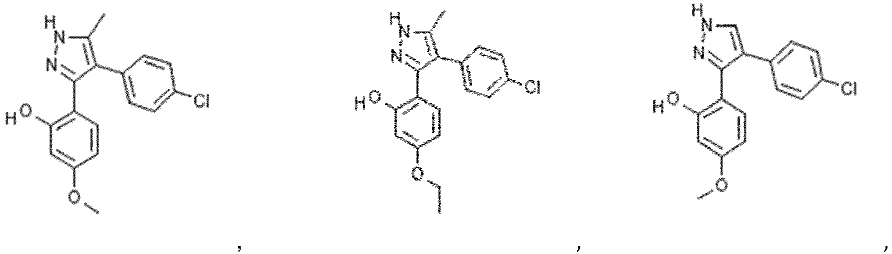 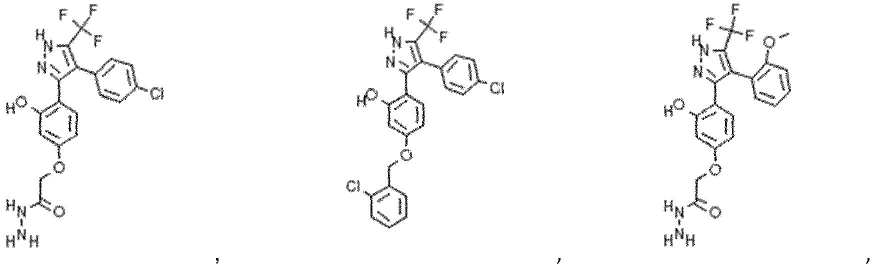 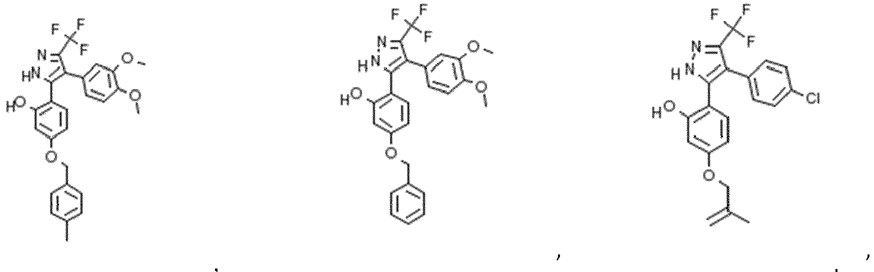 and

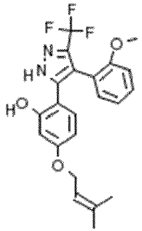

6. Use according to claims 1-5, wherein the use is as an antibacterial agent against gram- negative and/or gram-positive bacteria

7. Use of a compound of formula 1 as defined in claims 1-6 as a medicament against a pathogen.

8. Use according to claim 7, wherein the medicament is an antibacterial or an antibiotic medicament.

9. Use according to claims 1-8, wherein the pathogen is selected from the group consisting of *Staphylococcus, Escherichia, Streptococcus, Klebsiella, Mycobacterium, Pseudomonas, Clostridium, Acinetobacter, Enterococcus and Pasteurella. Bacillus anthracis, Listeria monocytogenes, Corynebacterium diphtheriae, Salmonella, Shigella, Moraxella, Helicobacter, Legionella, Vibrio, Rickettsia, Neisseria, Actinobacter, preferably Staphylococcus, Escherichia, Streptococcus, Mycobacterium, Pseudomonas, Enterococcus, and Pasteurella.*

10. Use according to claim 1-9, wherein the pathogen is selected from the group consisting of *Staphylococcus aureus Newman, Streptococcus suis, Mycobacterium marinum, Pasteurella multocida, Escherichia coli,* and *Enterococcus faecium.*

11. Use according to claim 1-10, wherein the pathogen is selected from the group consisting of *Staphylococcus aureus Newman, Streptococcus suis, Mycobacterium marinum, Pasteurella multocida, Escherichia coli,* and *Enterococcus faecium,* and the compound is selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-(4-bromobenzyl)oxy-2-[4-(4-chlorophenyl)-5-(trifluoromethyl)-1∼{H}-pyrazol-3-yl]phenol, 5-ethoxy-2-[4-(2-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 5-benzoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 5-(4-bromobenzyl)oxy-2-[4-(4-chlorophenyl)-5-(trifluoromethyl)-1∼{H}-pyrazol-3-yl]phenol, 2-[4-(4-chlorophenyl)-5-methyl-1∼H}-pyrazol-3-yl]-5-ethoxy-phenol, 2-[4-(4-chlorophenyl)-1∼{H}-pyrazol-5-yl]-5-methoxy-phenol, 5-(2-chlorobenzyl)oxy-2-[4-(4-chlorophenyl)-5-(trifluoromethyl)-1∼{H}-pyrazol-3-yl]pheno, 2-[3-hydroxy-4-[4-(2-methoxyphenyl)-5-(trifluoromethyl)-1∼{H}-pyrazol-3-yl]phenoxy]acetohydrazide, 2-[4-(3,4-dimethoxyphenyl)-5-(trifluoromethyl)-1∼{H}-pyrazol-3-yl]-5-(4-methylbenzyl)oxy-phenol, 2-[4-(4-chlorophenyl)-5-(trifluoromethyl)-1∼{H}-pyrazol-3-yl]-5-(2-methylallyloxy)phenol, 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol, preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-chlorophenyl)-3-(trifluoromethyl)isoxazol-5-yl]resorcinol, 4-[4-(4-bromophenyl)-3-methyl-isoxazol-5-yl]resorcinol.

12. Use according to claim 11, wherein the pathogen is selected from the group consisting of *Staphylococcus aureus Newman,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-(4-bromobenzyl)oxy-2-[4-(4-chlorophenyl)-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 5-benzoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, preferably selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol.

13. Use according to claim 11, wherein the pathogen is selected from the group consisting of *Streptococcus suis,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 2-[4-(4-chlorophenyl)-5-methyl-1∼{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol, preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol.

14. Use according to claim 11, wherein the pathogen is selected from the group consisting of *Pasteurella multocida,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 2-[4-(4-chlorophenyl)-5-(trifluoromethyl)-1∼{H}-pyrazol-3-yl]-5-ethoxy-phenol, 2-[4-(4-chlorophenyl)-5-methyl-1∼{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol.

15. Use according to claim 11, wherein the pathogen is selected from the group consisting of *Mycobacterium marinum,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-methyl-1∼{H}-pyrazol-3-yl]-5-ethoxy-phenol, 2-[4-(4-chlorophenyl)-1∼{H}-pyrazol-5-yl]-5-methoxy-phenol, 4-[4-(4-bromophenyl)-3-methyl-isoxazol-5-yl]resorcinol, preferably 2-[4-(3, 4-dimethoxyphenyl)-5-(trifluoromethyl)-1∼{H}-pyrazol-3-yl]-5-ethoxy-phenol.

16. Use according to claim 11, wherein the pathogen is selected from the group consisting of *Escherichia coli,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 5-ethoxy-2-[4-(2-methoxyphenyl)-5-(trifluoromethyl)-1∼{H}-pyrazol-3-yl]phenol, 5-methoxy-2-[4-phenyl-5-(trifluoromethyl)-1∼{H}-pyrazol-3-yl]phenol, 5-ethoxy-2-[4-phenyl-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-bromophenyl)-5-(trifluoromethyl)-1∼{h}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol, preferably selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 4-[4-(4-chlorophenyl)-3-(trifluoromethyl)isoxazol-5-yl]resorcinol, 4-[4-(4-bromophenyl)-3-methyl-isoxazol-5-yl]resorcinol.

17. Use according to claim 11, wherein the pathogen is selected from the group consisting of *Enterococcus faecium,* and the compound is selected from the group consisting of 2-[4-(3,4-dimethoxyphenyl)-5-(trifluoromethyl)-1∼{H}-pyrazol-3-yl]-5-ethoxy-phenol, 2-[4-(4-bromophenyl)-5-methyl-1∼{H}-pyrazol-3-yl]-5-methoxyphenol, 2-[4-(4-bromophenyl)-5-(trifluoromethyl)-1∼{H}-pyrazol-3-yl]-5-ethoxyphenol, 5-ethoxy-2-[4-(4-methoxyphenyl)-5-(trifluoromethyl)-1∼{H}-pyrazol-3-yl]phenol, 2-[3-hydroxy-4-[4-phenyl-5-(trifluoromethyl)-1∼{H}-pyrazol-3-yl]phenoxy]acetohydrazide, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 2-[4-(4-chlorophenyl)-5-methyl-1∼{H}-pyrazol-3-yl]-5-ethoxyphenol, 2-[4-(4-chlorophenyl)-1∼{H}-pyrazol-5-yl]-5-methoxy-phenol, 5-(2-chlorobenzyl)oxy-2-[4-(4-chlorophenyl)-5-(trifluoromethyl)-1∼{H}-pyrazol-3-yl]pheno, 2-[3-hydroxy-4-[4-(2-methoxyphenyl)-5-(trifluoromethyl)-1∼{H}-pyrazol-3-yl]phenoxy]acetohydrazide, 4-[4-(4-bromophenyl)-5-(trifluoromethyl)-1∼{H}-pyrazol-3-yl]resorcinol, 4-[4-(4-chlorophenyl)-3-(trifluoromethyl)isoxazol-5-yl]resorcinol, 4-[4-(4-bromophenyl)-3-methyl-isoxazol-5-yl]resorcinol preferably selected from the group consisting of 2-[4-(3, 4-dimethoxyphenyl)-5-(trifluoromethyl)-1∼{H}-pyrazol-3-yl]-5-ethoxy-phenol, 2-[4-(4-bromophenyl)-5-(trifluoromethyl)-1∼{H}-pyrazol-3-yl]-5-ethoxy-phenol, 5-ethoxy-2-[4-(4-methoxyphenyl)-5-(trifluoromethyl)-1∼{H}-pyrazol-3-yl]phenol, 2-[4-(4-chlorophenyl)-5-(trifluoromethyl)-1∼{H}-pyrazol-3-yl]-5-ethoxy-phenol, 5-(2-methylallyloxy)-2-[4-phenyl-5-(trifluoromethyl)-1∼{H}-pyrazol-3-yl]phenol, 4-[4-(4-bromophenyl)-3-methyl-isoxazol-5-yl]resorcinol.

18. Pharmaceutical composition comprising a compound as defined in any of claims 1-17.
